# DEMANDE DE BREVET EUROPEEN

(11) **EP 2 359 840 A1**
(43) Date de publication de la demande: **24.08.2011**
(21) Numéro de dépôt: 11151829.6
(22) Date de dépôt: 24.10.2001
(51) Int. Cl.: A61K 38/06

(54) **Utilisation d'inhibiteurs de l'apoptose des péricytes pour le traitement et/ou la prévention de la rétinopathie diabétique**

(30) Priorité: 24.10.2000 FR 0013640
(62) Demande divisionnaire de: 01982545.4
(71) Demandeur: Merck Sante, 69379 Lyon Cedex 08 (FR); INSERM (Institut National de la Santé et de la Recherche Médicale), 75654 Paris Cedex 13 (FR)
(72) Inventeur: Lecomte, Marc, 69380, Lissieu (FR); Denis, Ulriche, 69300, Caluire et Cuire (FR); Paget, Clarisse, 75011, Paris (FR); Wiernsperger, Nicolas, 69530, Orlienas (FR); Lagarde, Michel, 69150, Decines (FR)
(74) Mandataire: Novagraaf Technologies

(57) **Abrégé**

L'invention concerne l'utilisation d'un inhibiteur de l'apoptose des péricytes rétiniens pour la préparation d'un médicament utile pour prévenir ou traiter la rétinopathie diabétique, ledit inhibiteur de l'apoptose des péricytes rétiniens étant un inhibiteur de caspase, par exemple les peptides z-VAD-fmk, AEVD, z-VDVAD-fmk, z-DEVD-fmk ou z-LEHD-fmk.

## Description

La rétinopathie diabétique représente une des complications microvasculaires parmi les plus invalidantes du diabète, pouvant mener à son stade ultime à la cécité (Grange, 1995 ; Frank, 1995 ; Aiello LP et al , 1998). C'est la deuxième cause de cécité acquise dans les pays développés derrière la dégénérescence maculaire liée à l'âge (Nathan et al, 1991), et le risque d'un patient diabétique de devenir aveugle a été estimé 25 fois supérieur à celui de la population générale (Kahn et Hiller, 1974). Il n'y a pas, à l'heure actuelle, de traitement pharmacologique préventif ou curatif de cette complication, le seul traitement étant la photocoagulation rétinienne au laser ou la vitréotectomie dans les cas les plus sévères (Frank, 1995 ; Aiello, 1998).

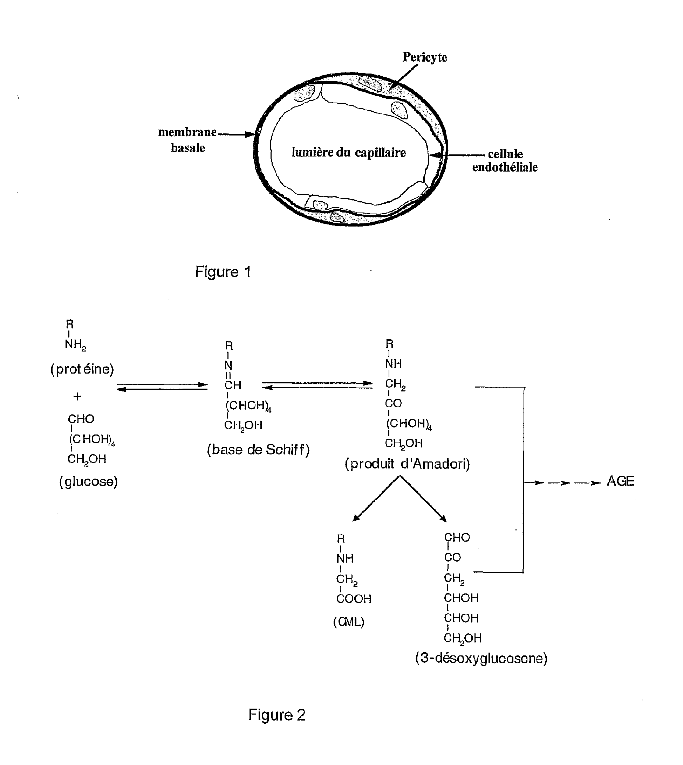

La rétinopathie diabétique est une complication diabétique évolutive, passant d'un stade dit "simple" ou débutant (background retinopathy), à une phase finale dite « rétinopathie proliférante », où il y a formation de néo-vaisseaux rétiniens, fragiles, menant à des hémorragies sévères, parfois avec décollement de la rétine, et à la perte de vision (Grange 1995, Frank, 1995). Dans la rétinopathie simple, les lésions microvasculaires se caractérisent par des microanévrysmes, petites hémorragies en points, exsudats et dilatations veineuses (Palmberg, 1977, ETDRS report n° 10, 1991). Cette forme de rétinopathie simple peut rester un grand temps cliniquement silencieuse. A ce stade de rétinopathie simple, des altérations cellulaires et structurales des capillaires rétiniens ont pu être notées, à partir de l'examen de rétines de patients diabétiques prélevées post mortem et comparées à des rétines d'individus normaux d'âges comparables. Comme montré sur la figure 1 en annexe, les capillaires rétiniens sont tapissés de cellules endothéliales du côté luminal du vaisseau, et de péricytes (ou cellules murales) situés à l'extérieur et enfouis dans la membrane basale du vaisseau.

Dans la rétine humaine ou la rétine de rat, le rapport numérique des péricytes aux cellules endothéliales est de un pour un (Kuwabara and Cogan, 1963). Les altérations observées à ce stade précoce consistent en un épaississement de la membrane basale des capillaires (Friedenwald, 1950) et une disparition sélective des péricytes (Cogan et al, 1961, Kuwabara and Cogan, 1963), abaissant le rapport numérique des péricytes par rapport aux cellules endothéliales des capillaires rétiniens à 0,3 pour 1 dans la situation pathologique et même 0,1 pour les stades ultimes (Cogan et al, 1961 ; Kuwabara and Cogan, 1963).

Les travaux réalisés à partir de rétines humaines prélevées *post mortem* chez des patients diabétiques de longue durée ont permis de montrer que les péricytes mourraient par apoptose, la mort cellulaire programmée, et non par nécrose, la mort brutale observée suite à une atteinte toxique (Mizutani et al, 1996, Li et al, 1997, Podesta et al, 2000). Mais la(les) voie(s) de signalisation(s) intra cellulaire(s) par la(es)quelle(s) ils disparaissent n'est (ne sont) pas connue(s).

La détection des péricytes apoptotiques a été effectuée *in situ,* sur les rétines entières par une technique de marquage des noyaux des cellules qui entrent en apoptose, la méthode TUNEL (terminal deoxynucleotidyl transferase mediated dUDP nick-end labeling) (Mizutani et al, 1996). Venant à l'appui de ces données, certains péricytes qui entrent en apoptose sont aussi colorés quand on utilise un anticorps dirigé contre une protéine impliquée dans l'apoptose qui se nomme Bax (Podesta et al, 2000).

Une autre équipe, après purification des péricytes de rétines de patients diabétiques, a montré une augmentation de l'expression du gène d'une autre protéine impliquée dans l'apoptose, la caspase 3 ou CPP 32 (Cysteine Protease Protein 32 kDa), en détectant un taux augmenté ARNm de cette protéine par une technique d'amplification par polymérisation en chaîne à la transcriptase inverse (Li et al, 1999). Ces péricytes purifiés voient aussi l'expression augmentée d'autres gènes impliqués dans les défenses antioxydantes cellulaires comme celui de la glutathion peroxydase par exemple, suggérant que le stress oxydant pourrait être impliqué dans la disparition des péricytes rétiniens par apoptose (Li et al, 1999).

Les mécanismes cellulaires sous-jacents par lesquels les péricytes meurent par apoptose sont encore largement inconnus. Les travaux réalisés dans le cadre de la présente invention ont précisément visé à identifier la chaîne d'événements menant à l'apoptose des péricytes de façon à définir des cibles pharmacologiques permettant d'enrayer le processus de perte des péricytes observée dans la phase précoce de la rétinopathie diabétique.

Des études entreprises dans le passé par The Diabetes Control Complications Trial Research Group (DCCT) (1993) ou l'UK Prospective Diabetes Study Group (UKPDS) (1998a et b) ont déjà montré le rôle clef du contrôle de l'hyperglycémie dans le développement de la rétinopathie diabétique. Plusieurs études ont déterminé que le glucose, à des concentrations pathologiques, pouvait induire des changements biochimiques susceptibles d'altérer les fonctions physiologiques ou la viabilité des cellules microvasculaires rétiniennes. L'effet inhibiteur du glucose *in vitro* sur la prolifération des péricytes, alors que la croissance des cellules endothéliales n'est pas modifiée (Porta et al, 1994), pourrait par exemple expliquer la disparition sélective des péricytes au cours de la rétinopathie. De même, la synthèse de composants de la membrane basale (collagène, laminine, fibronectine) stimulée dans les péricytes et les cellules endothéliales rétiniennes cultivés en présence de glucose (Li et al, 1984, Mandarino et al;, 1993) pourrait entraîner l'épaississement de la membrane basale.

Un autre mécanisme par lequel le glucose peut mener aux complications vasculaires dans le diabète, est la production et l'accumulation accrues de produits avancés de glycation ou AGE (Advanced Glycation End products) formés par la glycosylation non-enzymatique -ou glycation- de protéines, ADN ou lipides (réaction de Maillard, 1912) qui ont été mis en évidence dans de nombreuses études lors du diabète (Thornalley, 1999). La quantité d'AGE mesurée dans la peau de patients diabétiques est d'ailleurs fortement corrélée avec la sévérité des complications vasculaires (Beisswenger et al., 1995). La glycation, ou voie de Maillard représentée à la figure 2 en annexe, décrit la liaison des sucres réducteurs aux protéines: un sucre réducteur, sous forme ouverte, réagit d'abord avec le groupe amine libre d'acides aminés basiques contenus dans les protéines (lysine, arginine), entraînant la formation d'une base de Schiff stabilisée par la suite en produit d'Amadori.

Ces étapes sont réversibles et dépendantes de la concentration en substrats (protéines et sucres). Une fois formé, le produit d'Amadori subit une série de modifications qui mène soit à une fragmentation oxydative et à la formation de produits dits de glycoxydation comme la carboxyméthyllysine (CML), soit à la formation de dicarbonyles comme la 3-désoxyglucosone. Ces dicarbonyles, très réactifs, vont à leur tour réagir avec des amines de protéines, et propager ainsi la réaction de Maillard, en formant des ponts intra et intermoléculaires dans certaines protéines à durée de vie longue (Thornalley, 1999). D'autres voies de synthèse des AGE, outre le glucose ont été proposées récemment.

Le méthylglyoxal, formé par la fragmentation des trioses phosphates et l'oxydation de l'acétone dans le foie (grâce à des monooxygénases), est un autre dicarbonyle dont la concentration sanguine est augmentée chez les diabétiques (Mc Lellan et al., 1994) et qui, *in vitro,* dans des conditions physiologiques de pH et de température, est capable de modifier de manière irréversible, notamment les résidus arginine de protéines (Lo et al., 1994). De plus, les AGE de protéines formés après réaction avec le méthylglyoxal sont décrits comme des produits majeurs observés lors du diabète (Degenhardt et al, 1998). La formation d'AGE de protéines intracellulaires après réaction avec le méthylglyoxal, plus réactif que le glucose dans la réaction de Maillard, semble être une voie de formation majoritaire dans les cellules (Nishikawa et al., 2000, Shinohara et al, 1998).

Dans les microvaisseaux rétiniens, une accumulation de produits d'Amadori, précurseurs d'AGE (Schalkwijk et al, 1999), ainsi qu'une accumulation d'AGE a été mise en évidence grâce à des anticorps anti-AGE (ou anti-Amadori), au niveau de la membrane basale des péricytes et des cellules endothéliales (Stitt et al., 1997). Plusieurs études réalisées *in vitro* suggèrent que les AGE pourraient être impliqués dans le dysfonctionnement vasculaire rétinien. Les AGE modifient en effet la prolifération des deux types cellulaires, ils inhibent en particulier la croissance des péricytes (Chibber et al., 1997 ; Ruggiero-Lopez et al., 1997, Yamagishi et al, 1995). Les AGE extracellulaires sont capables de se lier à des récepteurs membranaires spécifiques et d'induire des réponses cellulaires variées. Des travaux ont montré qu'il y a colocalisation des AGE et des récepteurs aux AGE dans les microvaisseaux rétiniens de rats diabétiques (Stitt et al, 1997, Soulis et al, 1997). Ces récepteurs ont été identifiés sur plusieurs types cellulaires, dont les péricytes (Chibber et al, 1997, Yamagishi et al, 1995, Stitt et al, 1997, Thornalley, 1998 ) et différents types de récepteurs ont été isolés: p60, p90 (Yang et al., 1991), RAGE (Receptor for AGE), qui est un récepteur de la superfamille des immunoglobulines (Neeper et al, 1992) complexé à la lactoferrine (Schmidt et al, 1992) et la galectine 3 (Vlassara et al., 1995). RAGE, le récepteur le mieux caractérisé et qui est présent sur les péricytes (Yamagishi et al, 1995, Brett et al, 1993), après avoir fixé un AGE, induit un stress oxydant intracellulaire (Yan et al., 1994) et l'activation du facteur de transcription NF-κB (Yan et al., 1994) ainsi que de l'activation de p21 ras et de la MAP (Mitogen Activated Protein) - kinase (Lander et al., 1997) dans les cellules endothéliales de veine de cordon ombilical. L'activation du facteur de transcription NF-κB dans ces cellules peut être inhibée par divers antioxydants comme l'acide lipoïque (Bierhaus et al., 1997), les enzymes antioxydantes (Yan et al., 1994). Le trolox, un antioxydant, est capable de prévenir la perte des péricytes dans la rétine de rats diabétiques (Ansari et al, 1998) suggérant que le stress oxydant pourrait être impliqué dans leur disparition. Un ARN antisens dirigé contre le récepteur RAGE corrige l'effet antiprolifératif des AGE observé sur les péricytes (Yamagishi et al, 1995).

L'importance de la voie de Maillard et de l'accumulation des AGE dans la pathogenèse des complications diabétiques, et de la rétinopathie en particulier, a pu être explorée notamment grâce à l'utilisation d'inhibiteurs de glycation comme l'aminoguanidine, qui par son amine libre piège le glucose ou les dicarbonyles réactifs en les empêchant de réagir avec les protéines. Dans un modèle de rats diabétiques à la streptozotocine, l'aminoguanidine prévient l'accumulation des AGE dans les microvaisseaux rétiniens, ainsi que la disparition des péricytes et réduit de 80% le nombre de capillaires acellulaires dans la rétine (Hammes et al., 1991).

Les mécanismes par lesquels les AGE mènent à la disparition des péricytes, ainsi que le mode de disparition, apoptose ou nécrose, des péricytes par les AGE, restent des questions par contre non-résolues à ce jour et ont fait précisément l'objet d'une partie des travaux réalisés dans le cadre de la présente invention. Les Inventeurs se sont ainsi intéressés à élucider les voies de signalisation intracellulaires par lesquelles les AGE provoquent la mort des péricytes par apoptose.

Ces travaux ont permis de concevoir de nouvelles méthodes pour prévenir ou traiter la rétinopathie diabétique consistant à administrer à un sujet une quantité efficace d'au moins un inhibiteur de l'apoptose des péricytes rétiniens. L'invention est remarquable en ce qu'elle offre de nouveaux moyens de traitement ou de prévention tant d'une rétinopathie établie cliniquement que d'une rétinopathie simple. Les signes cliniques d'une rétinopathie simple sont généralement l'apparition d'un ou deux microanévrysmes par fond d'oeil. On désignera ci-après par rétinopathie diabétique, tant une rétinopathie établie qu'une rétinopathie simple.

Ainsi, l'invention a pour objet l'utilisation d'un inhibiteur de l'apoptose des péricytes rétiniens pour la préparation d'un médicament utile pour prévenir ou traiter la rétinopathie diabétique.

Il a été montré dans le cadre de l'invention que les AGE, produits après incubation de l'albumine avec du méthylglyoxal, ajoutés dans le milieu de culture des péricytes (2 µM) pendant deux passages (10-15 jours) provoquait une stimulation de leur mort par apoptose (3 fois le taux basal), ainsi que l'accumulation intracellulaire de céramides et de diacylglycérol (DAG) (200 % du taux basal). L'apoptose et la production accrue de céramides et DAG sont normalisées en présence d'antioxydants dans le milieu de culture, comme la Nα-acétyl-cystéine (NAC) à 10 ou 50 µM, concentrations non cytotoxiques, suggérant que les AGE induisent un stress oxydant dans les cellules et qu'ils contribuent à leur mort par apoptose.

En conséquence, l'invention concerne à titre d'inhibiteur de l'apoptose des péricytes rétiniens, un composé anti-oxydant. A titre d'exemple de tels composés anti-oxydants on peut citer la Nα-acétyl-L-Cystéine, l'acide lipoïque, l'ebselen, la zéaxanthine, la coelentéramine, un dérivé ou un mélange de ceux-ci. Les composés anti-oxydants sont tout particulièrement intéressants car ils réduisent la production des céramides et/ou des DAG. Ainsi, l'invention vise également tout composé capable de réduire directement ou indirectement la production des céramides et/ou des DAG.

Il a en effet été mis en évidence que l'apoptose est corrigée par d'autres antioxydants que la N-acétyl-L-Cystéine comme l'acide lipoïque (7 et 14 µM) ou l'ebselen (5 et 10 µM), et partiellement (réduction de l'ordre de 50%) par la zéaxanthine (2 µM) ou la coelentéramine (2 µM), tous utilisés à des concentrations non cytotoxiques.

Il a en outre maintenant été trouvé que les DAG et les céramides sont produits par une activation couplée de la phospholipase C spécifique de la phosphatidylcholine (PC-PLC) et de la sphingomyélinase acide et que les céramides ne semblent pas être synthétisés *de novo.*

En effet, le 10,11-Dihydro-5-[3-(methylamino)propyl]-5H-dibenz[b,f]azepine aussi désigné désipramine (0,1 et 0,3 µM), qui est un inhibiteur de la sphingomyélinase acide, ou le tricyclo[5.21.0(2.6)]decyl-(9[8]xanthogenate aussi désigné D609 (9,4 ou 30 µM), qui est un inhibiteur de la PC-PLC, inhibent bien la production de céramides, mais seul le D609 inhibe à la fois la production de céramides (95%) et de DAG (80%), suggérant que la production de DAG par la PC-PLC est située en amont de l'activation de la sphingomyélinase acide produisant les céramides. La fumonisine B1 (0,01 µM), inhibiteur de la synthèse *de novo* des céramides, n'inhibe que marginalement l'apoptose induite par les AGE (20%) suggérant que cette voie de production des céramides n'est probablement pas impliquée. L'apoptose, quant à elle, n'est inhibée que 50% environ en présence de 10,11-Dihydro-5-[3-(methylamino)propyl]-5H-dibenz[b,f]azepine (désipramine) (0.3 µM) ou de tricyclo [5.21.0 (2.6) ] decyl- (9 [8]xanthogenate (D609) (30 µM), ou leur combinaison, alors que les antioxydants protègent totalement les cellules, suggérant une voie parallèle à celle de la PC-PLC et sphingomyélinase acide menant à l'apoptose des péricytes à partir du stress oxydant intracellulaire.

En conséquence, l'invention concerne tout particulièrement l'utilisation:
- d'un inhibiteur de la phosphatidylcholine-phospholipase C (PC-PLC) en tant qu'inhibiteur de la formation des DAG,
- d'un inhibiteur de la sphingomyélinase acide en tant qu'inhibiteur de la formation des céramides,
pour la préparation d'un médicament utile pour le traitement et/ou la prévention de la rétinopathie diabétique. En effet, ces inhibiteurs sont remarquables pour leur capacité à protéger les péricytes rétiniens de l'apoptose.

Parmi les inhibiteurs de la phosphatidylcholine-phospholipase C, on peut citer plus particulièrement le D609, ou un dérivé de celui-ci.

Parmi les inhibiteurs de la sphingomyélinase acide, on peut citer plus particulièrement la désipramine, ou un dérivé de celle-ci.

En outre, grâce à des inhibiteurs peptidiques spécifiques de protéases effectrices à cystéine impliquées dans l'apoptose, les caspases, les Inventeurs ont pu explorer l'intervention des caspases dans l'apoptose des péricytes par les AGE. Ces peptides sont modifiés chimiquement par un groupement benzyleoxycarbonyle du côté N-terminal (z) et par un groupe (O-méthyle)-fluorométhyle cétone (fmk) du côté C-terminal les rendant plus perméants aux cellules. Les peptides testés sur l'apoptose des péricytes provoquée par les AGE ont été:
- le tripeptide z-VAD-fmk, un inhibiteur de toutes les caspases,
- z-DEVD-fmk, un inhibiteur spécifique d'une caspase effectrice, la caspase-3,
- z-LEHD-fmk, un inhibiteur spécifique d'une caspase effectrice, la caspase-9,
- z-IETD-fmk, un inhibiteur spécifique d'une caspase initiatrice, la caspase-8,
- z-VDVAD-fmk et z-AEVD-fmk inhibiteurs respectivement de la caspase-2 et -10, initiatrices.

Le peptide z-VAD-fmk (pan caspase inhibiteur), z-DEVD-fmk (caspase-3), z-AEVD-fmk (caspase-10) et z-LEHD-fmk (caspase-9) inhibent tous les quatre l'apoptose induite par les AGE, mais seul le peptide z-VAD-fmk a inhibé la production de DAG et de céramides alors que z-DEVD-fmk et z-LEHD-fmk n'ont pas eu d'effet. Ceci suggère qu'une caspase initiatrice est impliquée en amont de la production de DAG et de céramides, et qu'elle est inhibée par z-VAD-fmk. De plus, deux caspases effectrices, la caspase-3 et la caspase-9, sont impliquées dans la phase finale de l'apoptose et sont inhibées par z-DEVD-fmk et z-LEDH-fmk, respectivement, et sont situées après la production de DAG et de céramides, qui n'est pas affectée par ces deux peptides.

Sur la base de la spécificité des inhibiteurs utilisés, la caspase initiatrice impliquée ne semble pas être la caspase-8, z-IETD-fmk n'ayant pas eu d'effet sur l'apoptose. La caspase-10, inhibée par z-AEVD-fmk, semble par contre impliquée, ce peptide inhibant totalement l'apotose des péricytes induite par les AGE. D'autre part, z-VDVAD-fmk inhibe partiellement l'apoptose suggérant que la caspase-2 initiatrice serait elle aussi impliquée. En résumé, ces travaux ont tout particulièrement mis en évidence que les caspase-2 ou 10 peuvent être impliquées, comme caspases initiatrices, dans la cascade menant à l'apoptose des péricytes induite par les AGE, et que la caspase-9 et la caspase-3 effectrices semblent être impliquées dans la phase finale de cette apoptose.

En conséquence, l'invention se rapporte tout spécialement à l'utilisation d'un inhibiteur de caspase pour la préparation d'un médicament protégeant les péricytes rétiniens de l'apoptose utile pour le traitement et/ou la prévention de la rétinopathie chez un patient diabétique.

De tels inhibiteurs sont de préférence de type peptidique. Il s'agit avantageusement de peptides modifiés chimiquement par un groupement benzyleoxycarbonyle du côté N-terminal (z) et par un groupe (O-méthyle)-fluorométhyle cétone (fmk) du côté C-terminal les rendant plus perméants aux cellules. A titre d'exemple de peptide inhibiteur de caspase ont peut citer le tripeptide z-VAD-fmk, un inhibiteur de toutes les caspases.

L'invention envisage tout particulièrement l'utilisation d'un inhibiteur d'une caspase initiatrice, de préférence choisie parmi les caspases 2 ou 10 et/ou d'un inhibiteur d'une caspase effectrice, de préférence choisie parmi les caspases 3, 6, 7 ou 9 tout préférentiellement des caspases 3 ou 9, pour la préparation d'un médicament utile pour prévenir ou traiter la rétinopathie diabétique.

A titre d'exemple d'inhibiteur de la caspase initiatrice 10, on peut citer le peptide z-AEVD-fmk.

A titre d'exemple d'un inhibiteur de la caspase initiatrice 2, on peut citer le peptide z-VDVAD-fmk.

A titre d'exemple d'un inhibiteur de la caspase effectrice 9, on peut citer le peptide z-LEHD-fmk.

A titre d'exemple d'inhibiteur de la caspase effectrice 3, on peut citer le peptide z-DEVD-fmk.

L'invention envisage tout particulièrement l'utilisation d'une association d'au moins deux inhibiteurs de l'apoptose des péricytes rétiniens cités précédemment pour la préparation d'un médicament utile pour le traitement ou la prévention de la rétinopathie diabétique. Ainsi, une forme de mise en oeuvre préférée de l'invention consiste à administrer conjointement à un patient une quantité efficace d'au moins deux composés choisis parmi:
- un composé anti-oxydant,
- un inhibiteur de la phosphatidylcholine-phospholipase C (PC-PLC),
- un inhibiteur d'une sphingomyélinase acide,
- un inhibiteur de caspase.

L'administration desdits inhibiteurs de l'apoptose des péricytes rétiniens peut être simultanée ou non. En conséquence, l'invention a également pour objet une composition pharmaceutique comprenant à titre d'agents actifs au moins un inhibiteur de l'apoptose de péricytes rétiniens décrits précédemment.

Ainsi, l'invention concerne plus particulièrement une composition comprenant à titre d'agent(s) actif(s) au moins un composé choisi parmi: un anti-oxydant, un composé inhibiteur de la production des céramides et/ou des DAG, un inhibiteur de la sphingomyélinase acide, un inhibiteur de la phosphatidylcholine-phospholipase C, un inhibiteur de caspase, comme définis précédemment, ou d'un mélange d'au moins deux de ceux-ci. En effet, une telle composition est particulièrement utile pour prévenir ou traiter la rétinopathie diabétique.

On peut citer à titre d'exemple préféré d'une telle composition, celles comprenant à titre d'agents actifs, un antioxydant et/ou un inhibiteur de la phosphatidylcholine-phospholipase C (PC-PLC) et/ou un inhibiteur de la sphingomyélinase acide, associé(s) dans ladite composition avec au moins un inhibiteur de caspase comme défini précédemment.

La composition selon l'invention peut accepter toute forme d'administration connue de l'homme du métier, par voie générale (p.o.), par injection sous-cutanée (s.c.) intra-péritonéale (i.p.) ou intra-veineuse (i.v.), dans une formulation adaptée dont le dosage en agent(s) actif(s) permet une administration journalières comprise préférentiellement entre environ 0,01 et environ 200 mg/kg de masse corporelle, préférentiellement entre 0,5 et environ 75 mg/kg de masse corporelle et idéalement entre environ 1 et 50 mg/kg de masse corporelle, mais pas limitées à celles-ci et pouvant aller plus haut en cas d'un composé peu toxique et peu actif in vivo. Pour l'administration autre qu'oculaire, la quantité de composé actif qui sera combinée à l'excipient pour l'administration en dose unique dans une composition ne comprenant qu'un composé actif défini précédemment, peut varier et dépend du mode d'administration. Une préparation typique contiendra de 5% environ à 95% de composé actif (w/w). Les préparations contiendront, de préférence, de 20% à 80% (w/w) environ de composé actif.

Dans le cas d'une composition selon l'invention comprenant au moins deux composés actifs définis précédemment, chacun d'eux est présent à des doses allant d'environ 10% à 80% des doses normalement administrées dans un régime de monothérapie comme décrit ci-dessus. Lors de l'amélioration de la condition d'un patient, une dose d'entretien d'un composé, d'une composition ou d'une combinaison concernée par cette invention peut être administrée, si nécessaire. De même, la dose ou la fréquence d'administration peut être réduite en fonction des symptômes, et si ceux-ci sont circonscrits à un niveau désiré, le traitement pourrait être interrompu. De la même manière, certains patients pourraient requérir un traitement intermittent sur le long-terme jusqu'à la récurrence de l'un ou l'autre symptôme de la rétinopathie.

Les formulations pour voie générale peuvent être très variées, comprimés, gélules, poudres, solutions, comprenant le(s) agent(s) actif(s), les excipients, masqueurs de goût, tampons, agents conservateurs, connus de l'homme du métier et pharmaceutiquement acceptables. Les formes injectables comprennent le(s) principe(s) actif(s) dans un solvant de préférence aqueux, éventuellement avec un co-solvant pouvant abaisser la constante diélectrique du milieu, contenant un couple de sels qui tamponne le pH à des valeurs proches des valeurs physiologiques (pH 7,0 -7,4) connus de l'homme du métier, et éventuellement un agent tensio-actif augmentant la solubilité du (des) principe (s) actif (s) .

Une forme d'administration toute préférée selon l'invention, consiste en une application topique ou une injection oculaire. Cette forme d'application permet de limiter les effets systémiques généraux négatifs que pourrait engendrer l'usage prolongé d'inhibiteurs de caspases administrés par voie générale. Une composition pharmaceutique pour une application topique oculaire selon l'invention peut être formulée dans un onguent (pommade, gel ou solution) convenable contenant le (les) composé(s) actif(s), dissous ou en suspension dans un véhicule.

Ainsi, à titre d'exemple, il peut s'agir de gouttes oculaires, dont les véhicules pharmaceutiquement acceptables peuvent être du chlorure de sodium isotonique (0,9%, 308 mOsm/L) ou des solutions salines isotoniques comprenant du chlorure de sodium, du chlorure de potassium, du gluconate de potassium, du chlorure de calcium, de l'acide borique, du borate ou borax de sodium, du lactate de sodium, de l'hydrogénophosphate de sodium dihydraté et du dihydrogénophosphate de sodium dodécahydraté, du sorbitol, avec ou sans agents mimant la viscosité lacrymale tels que de l'hypromellose 4000 cP, du dextran, et des agents conservateurs comme de l'édetate de sodium, du bromure de benzododécinium, du mercurothiolate sodique, du chlorure de benzalkonium, du parahydroxybenzoate de méthyle, du métabisulfite de sodium, du borate de phénylmercure. Ces composés sont donnés à titre d'exemples non limitatifs de réalisation de l'invention.

Dans le cas d'une pommade ou d'un gel ophtalmique, des polymères variés peuvent être utilisés, comme par exemple, le carbomère 980 NF (polymère de 4.10⁶ Da), le sorbitol, une huile par exemple de paraffine, le monostéarate de sorbitan, le polysorbate 60, la cire de cétyl esters, la cire de cetéaryl esters, la vaseline, le méthylcellulose, une graisse. D'autres principes émulsifiants connus de l'homme du métier peuvent rentrer dans ces compositions.

Une telle composition selon l'invention comprend par exemple entre 0,01 et 10% et de préférence entre 0,01 et 4% en poids desdits inhibiteurs, afin de limiter tout risque de réactions indésirables à long terme, et un passage systémique trop important pouvant mener à l'arrêt du traitement topique. Une administration typique selon l'invention est une administration dans le cul-de-sac conjonctival, par exemple une fois et jusqu'à quatre fois par jour, avec une préférence pour une ou deux applications journalières.

Un système d'administration de drogues de type Ocusert (Friederich, 1974), formé d'un réservoir de forme ellipsoïdale en gel de polyacrylate, butylcyanoacrylate ou pluronique F127 (Desai et Blanchard, 1998) ou encore de fibrine ou collagène (Rubin et al, 1973), par exemple, contenant un ou plusieurs des composés définis précédemment, maintenu entre deux couches de copolymères (méthylcellulose 15 cP ou hydroxypropylcellulose 80-120 cP (Desai et Blanchard, 1998)), contrôlant l'administration du ou desdits composés dans le temps, et placé dans le cul-de-sac conjonctival, fait aussi partie de la présente invention. Un morceau d'éponge de 5x2 ou 7x4 mm (Altomed^{™}, mais aussi Weck^{™} ou Merocel^{™}) imbibé par la solution contenant les composés actifs et placé pendant quelques minutes dans le cul-de-sac conjonctival peut aussi être envisagé pour l'application topique des produits (Wilkins et al, 2000).

Les compositions selon l'invention peuvent être aussi formulées dans des émulsions, où les composés définis précédemment sont encapsulés dans des goutelettes de l'ordre du micron (liposomes), par exemple dans de l'huile ou de la lécithine de soja, la gélatine (Vandervoort et Ludwig, 1999), la viscosité de la préparation peut être augmentée par l'addition de méthylcellulose ou de carmellose de sodium (Zurowska-Pryczkowska, 1999). Les composés peuvent aussi être encapsulé dans des hydroxypropyl-β-cyclodextrines (solution à 5%, par exemple) de concentration variable mais pouvant aller aussi bas qu'un rapport molaire de un pour un par rapport au(x) principe(s) actif(s), ce qui augmenterait leur(s) biodisponibilité(s), leur(s) stabilité(s), et leur(s) solubilité(s) (Freedman et al, 1993).

L'invention concerne aussi l'utilisation d'oligonucléotides antisens complémentaires d'une partie d'une séquence polynucléotidique choisie parmi celle codant pour une caspase, la phosphatidylcholine-phospholipase C, la sphingomyélinase acide pour la préparation d'un médicament destiné au traitement et/ou à la prévention de la rétinopathie diabétique.

En effet, les oligodéoxynucléotides phosphorothioates antisens offrent l'opportunité de moduler l'expression génique de gènes spécifiques et sont porteurs d'un potentiel énorme pour une application clinique. Différents oligoméres antisens de tailles différentes, entre 6 et 25 nucléotides de longueur, dirigés contre la caspase 2, 3, 9 ou 10 ou contre la PC-PLC, ou encore contre la sphingomyélinase acide sont synthétisés et utilisés seuls ou en combinaisons.

Ces oligonucléotides antisens sont définis dans les séquences des caspases proches du N- ou C-terminus de ces enzymes, pour éviter les réactions croisées et être le plus spécifique possible. Les oligonucléotides de PC-PLC sont définis soit dans la partie C-terminale ou dans la partie N-terminale, proche d'histidines conservées dans le site actif, telles que H69 et H118 de la protéine de Listeria monocytogenes (Zuckert WR et al, 1998).

Des oligonucléotides de sphingomyélinase acide sont définis dans la partie N-terminale ou C-terminale de l'enzyme humaine (Schuchman et al., 1991) pour être le plus spécifique possible et éviter les réactions croisées.

Les oligonucléotides sont mélangés à une concentration comprise entre 1 et 10 µM en présence d'un copolymère de polyoxyéthylène-polyspermine ou de microsphères de fibrinogène (Oganesian et al, 1999), sur un morceau de membrane de polytétrafluoroéthylène. (PTFE) (Karesh, 1987) ou de polyuréthane (Nuyts et al, 1999), et ce patch peut alors être appliqué à l'intérieur de l'oeil, dans la cavité vitréenne et dans l'espace situé sous la rétine par une technique microchirurgicale du *pars plana* (l'espace de 2 mm situé à 4-5 mm latéralement de la jonction cornéo-sclérale) (Algvere et al, 1999) et éventuellement maintenu en place à l'aide d'une colle ciment à base de N-butylcyanoacrylate (Nexacryl^{™}) (Leahey et al, 1993), afin de permettre une diffusion contrôlée lente et étalée dans le temps de ces inhibiteurs.

Les oligonucléotides antisens peuvent être aussi injectés tels quels, ou encore encapsulés dans des liposomes, après émulsion avec différentes graisses ou huiles, qui sont ensuite recouverts de l'enveloppe virale du virus hémaglutinant du Japon inactivé (virus de Sendai) après fusion, et ensuite injecté dans l'espace sous rétinien par le pars plana (Hangai et al, 1998). Les doses injectées, pour être actives, se situent avantageusement dans une fourchette comprise entre 10 et 200 µg par oeil (Leeds et al, 1998).

L'invention envisage également la mise en oeuvre d'une stratégie de gène antisens pour les caspases-2, 3, 9 ou 10 dans une méthode de traitement et/ou de prévention de la rétinopathie diabétique. En effet, les gènes antisens de ces caspases sont introduits dans un adénovirus, comme indiqué ci-dessous, qui est injecté dans l'espace sous rétinien, après injection par le pars plana comme décrit ci-dessus (Lai et al, 2000).

L'invention envisage encore l'utilisation de peptides cycliques ou linéaires, de deux ou trois acides aminés à cinq ou six, inhibiteurs compétitifs des caspases, pour la préparation d'un médicament destiné au traitement et/ou à la prévention de la rétinopathie diabétique.

Il s'agit par exemple de peptides tels que, VAD, inhibiteur général de caspases, DEVD, inhibiteur spécifique de caspase 3, AEVD, inhibiteur spécifique de caspase 10, VDVAD, inhibiteur spécifique de caspase 2, ou LEHD, inhibiteur spécifique de caspase-9. Ces peptides linéaires sont protégés chimiquement du côté N-terminal par un dérivé d'un groupement acétyle ou benzyle, tel que mais pas limité à un groupement benzyleoxycarbonyle (z ou BOC), et du côté C-terminal par un groupement méthyl cétone, tel que mais pas limité à un groupement fluoro- ou chloro-méthylcétone ou encore à un groupement 2,6 diméthyl ou dichloro benzoyloxyméthylcétone, et dont les chaînes latérales d'acides aminés acides aspartiques (D) sont O-méthylées. Ces peptides peuvent aussi être couplés à des peptides de 16 acides aminés provenant de la séquence hydrophobe du peptide signal du facteur de croissance des fibroblastes (FGF) de Kaposi, améliorant leur perméabilité aux cellules (NH₂-AAVALLPAVLLALLAP-COOH). Des dérivés de l'aspartate seul, protégé par un groupement benzyle et un groupement fluorométhyl cétone sont aussi envisageables dans cette invention. Ces peptides pouvant être administrés par injection, par voie s.c., i.p. ou i.v., ou en application topique dans le cul-de-sac conjonctival, préférentiellement par voie i.v. ou topique, et dans une formulation qui permette une infusion lente et contrôlée pour réduire la fréquence des injections dans le cadre d'un traitement préventif. De telles solutions existent et sont constituées de préparations de polymères biodégradables contenant le peptide et contrôlant sa diffusion, tel que, mais pas limité à, un poly(DL-lactide-co-glycolide) (PLGA), injectable sous forme (a) soit de microsphères ayant approximativement de 1 à 100 µm de diamètre, ou (b) un implant unique, cylindrique, de taille approximative entre 0,8 mm et 1,5 mm de diamètre, appelé un "minicylindre" (Zhu et al, 2000). La stabilisation des peptides incorporés en présence d'un anti-acide tel que Mg(OH)₂ lors de l'encapsulation dans le polymère est aussi envisagée dans le cadre de la présente invention. (Zhu et al, 2000).

L'invention concerne aussi l'utilisation d'une molécule d'acide nucléique comprenant au moins une séquence polynucléotidique codant pour un peptide de 2 à 10 acides aminés inhibiteur compétitif d'une caspase pour la préparation d'un médicament destiné au traitement et/ou à la prévention de la rétinopathie diabétique.

En effet, l'invention envisage également une méthode de traitement et/ou de prévention de la rétinopathie diabétique consistant à produire localement et en continu au niveau intravitréen et sub-rétinien, *in situ,* ces petits peptides. Il s'agit alors avantageusement d'une stratégie d'expression génique de ces petits peptides inhibiteurs de caspases, dans le cadre d'un protocole de thérapie génique avec les vecteurs couramment utilisés lors d'une telle approche. Par exemple, les oligoméres de désoxyribonucléotides correspondant aux séquences peptidiques de VAD, DEVD, VDVAD ou AEVD, peuvent être synthétisés par des machines automatiques accessibles à l'homme de l'art. Dans le cas de AEVD, il s'agirait de produire deux oligomères complémentaires de séquences suivantes, l'oligomère codant, 5' - G A T C C (G ou A) (T, C, A ou G) (T, C, A ou G) A T G G C (T, C, A ou G) G A (A ou G) G T (T, C, A ou G) G A (T ou C) T A A G -3' et le non-codant 5' - A A T T C T T A (A ou G) T C (T, C, A ou G) A C (T ou C) T C (T, C, A ou G) G C C A T (T, C, A ou G) (T, C, A ou G) G -3'. Les deux oligomères une fois synthétisés sont ensuite phosphorylés par la kinase du bactériophage T4, et hybridés, pour obtenir la séquence d'ADN double brin correspondante à la séquence peptidique AEVD, qui peut être alors clonée dans le site polylinker contenant les sites de coupures d'enzymes de restriction adéquats (BamHI et EcoRI, dans le cas décrit) d'un vecteur d'expression de type pAC, qui peut alors produire des adénovirus recombinants exprimant le petit peptide désiré par la stratégie suivante. Le vecteur pACCMVpLpA est produit par insertion du promoteur/enhancer de gènes précoces constitutif du cytomégalovirus (CMV), le polylinker (pL) de sites de restriction du vecteur pUC 18, et un fragment du génome du virus simien SV 40 qui inclus l'antigène petit - T (t) et le signal de polyadénylation (pA), dans le vecteur pAC (Gluzman et al, 1982). L'ADN double brin correspondant à la séquence AEVD est alors introduite dans le vecteur linéarisé avec les enzymes de restriction correspondant aux sites de restriction flanquant la séquence AEVD, dans l'exemple décrit BamH1 et Eco R1. Le plasmide correspondant est ensuite produit par des techniques classiques de clonage bactérien, et l'insertion vérifiée par séquençage en utilisant la séquence flanquante du polylinker. Le plasmide résultant est ensuite co-transfecté avec pJM17 (McGrory et al, 1988) dans des cellules 293 (cellules embryonnaires rénales humaines transfectées par l'adénovirus AdEA) (Graham et al., 1977) par coprécipitation d'ADN avec le phosphate de calcium. pJM17 code pour un adénovirus 5 de séquence génomique complète interrompu par un plasmide bactérien pBRX à la position 3.7 unité de la carte génétique de l'adénovirus, excédant donc les limites d'empaquetage pour l'adénovirus. La recombinaison homologue entre le plasmide recombinant pACCMVpLpA et pJM17 dans les cellules 293 génère un génome de taille empaquetable, dans lequel la région précoce 1 est remplacée par le gène chimérique cloné, rendant le virus recombinant défectif pour la réplication. Le virus résultant est nommé AdCMV-AEVD. La présence de l'insertion correspondant au peptide AEVD est confirmée par PCR en utilisant des primers flanquant la séquence AEVD et en séquençant le produit de PCR obtenu. Les plaques virales appropriées sont alors amplifiées en stocks contenant 5-50 x 10⁷ plaque forming unit (pfu) /ml et stockées dans le DMEM supplémenté avec 10% de sérum de veau foetal. L'adénovirus recombinant peut alors être utilisé et encapsulé, par une des techniques décrites ci-dessus et injecté par le pars-plana dans l'espace sub-rétinien dans la cavité intravitréenne, afin d'avoir *in situ* une production soutenue et étalée dans le temps d'inhibiteurs de caspases, permettant de prévenir la perte des péricytes par apoptose.

L'invention se rapporte enfin à une méthode d'identification de composés capables de prévenir ou traiter la rétinopathie diabétique, caractérisée en ce qu'elle comprend la mise en contact d'une culture de péricytes traités par des AGE avec un composé à tester et en ce que l'on évalue l'apoptose dans lesdits pérycites pour déterminer la capacité dudit composé à prévenir ou traiter la rétinopathie diabétique. Avantageusement, cette évaluation comprend la comparaison avec l'apotose d'une culture de péricytes non traités par ledit composé et/ou non traités par des AGE.

Le composé testé est choisi parmi ceux envisagés précédement et notamment dans le groupe comprenant: les anti-oxydants, les inhibiteurs de la production des céramides et/ou des DAG, les inhibiteurs de caspase, les oligonucléotide antisens, de 6 à 25 nucléotides, complémentaire d'une partie d'une séquence polynucléotidique choisie parmi celle codant pour une caspase, la phosphatidylcholine-phospholipase C, la sphingomyélinase acide, les molécules d'acide nucléique comprenant au moins une séquence polynucléotidique codant pour un peptide, de 2 à 10 acides aminés, inhibiteur compétitif d'une caspase.

D'autres avantages et caractéristiques de l'invention apparaîtront de la description qui suit rapportant:
1) Les effets des AGE-méthylglyoxal sur les péricytes rétiniens de boeuf.
2) L'induction d'un stress oxydant par les AGE-méthylglyoxal dans les péricytes rétiniens en culture.
3) Les voies de production des céramides et des DAG activées dans les péricytes rétiniens en réponse aux AGE-méthylglyoxal.
4) L'implication des caspases.

Il sera fait référence dans ce qui suite aux dessins en annexe dans lesquels:
La figure 1 représente le schéma d'un capillaire rétinien.
La figure 2 représente la voie de Maillard.
La figure 3 représente le marquage au ³²P des céramides et des DAG.
La figure 4 représente l'effet des AGE-méthylglyoxal sur l'apoptose des péricytes rétiniens de boeuf.
La figure 5 représente l'effet des AGE-méthylglyoxal sur la production de céramides et de DAG dans les péricytes rétiniens de boeuf.
La figure 6 représente l'effet de la Nα-acétyl-L-Cystéine sur l'apoptose induite des BRP par les AGE-méthylglyoxal.
La figure 7 représente l'effet de la Nα-acétyl-L-Cystéine sur la production de céramides et de DAG dans les BRP induite par les AGE-méthylglyoxal.
La figure 8 représente les effets de divers antioxydants sur l'apoptose des péricytes en réponse aux AGE-méthylglyoxal.
La figure 9 représente l'effet de la fumonisine B1 sur l'apoptose des péricytes induite par les AGE-méthylglyoxal.
La figure 10 représente l'effet de la désipramine sur l'apoptose des péricytes induite par les AGE-méthylglyoxal.
La figure 11 représente l'effet de la désipramine sur la production stimulée de céramides et de DAG dans les BRP induites par les AGE-méthylglyoxal.
La figure 12 représente la voie de production des céramides et des DAG dans les péricytes envisagée sur la base de l'invention.
La figure 13 représente l'effet du D609 sur l'apoptose des péricytes induite par les AGE-méthylglyoxal après deux passages.
La figure 14 représente l'effet du D609 sur la production stimulée de céramides et de DAG dans les BRP induites par les AGE-méthylglyoxal.
La figure 15 représente la voie de signalisation menant à l'apoptose des péricytes envisagée sur la base de l'invention.
La figure 16 représente l'effet des peptides inhibiteurs de caspases sur l'apoptose des péricytes induite par les AGE-méthylglyoxal.
La figure 17 représente l'effet des peptides inhibiteurs de caspases z-VAD-FMK, z-LEHD-fmk et z-DEVD-FMK (50 µM) sur la production stimulée de céramides et de DAG dans les BRP induites par les AGE-méthylglyoxal.
La figure 18 représente la voie de signalisation menant à l'apoptose des péricytes envisagée sur la base de l'invention.
La figure 19 est un wester blot avec un anticorps anti-caspase 10 humaine sur des lysats cellulaires de péricytes traités ou non avec AGE.

### PARTIE A: MATERIEL ET METHODES.

### I - Matériel.

### 1) Réactifs.

Tous les produits pour la culture cellulaire proviennent de Sigma (France) sauf le SVF (Gibco, France), la collagénase IA et la collagénase/dispase (Boehringer-Mannheim, France). Tous les autres réactifs sont purs « pour analyse » et ont été achetés chez Sigma (France), sauf les plaques de silice (silica gel 60 G, Merck), la DAG kinase et les céramides (Tebu, France). Les colonnes PD-10 proviennent de Pharmacia (Suède). Le kit de marquage à l'annexine-V provient de Boehringer-Mannheim (France). La N-acétyl-L-cystéine, la désipramine, l'acide lipoïque et l'ebselen proviennent de Sigma (France). Les peptides inhibiteurs de caspases proviennent de Calbiochem (France), la fumonisine B1 et le D609 proviennent de Biomol (France).

Préparation des solutions d'agents pharmacologiques:
Les solutions sont stérilisées au moyen d'un filtre acrodisc stérile 0,2 µm (Gellman Science).
La solution mère de 1 µM de N-acétyl-L-cystéine est préparée extemporanément: 163,2 mg sont repris dans 1 ml de DMEM puis la solution est filtrée stérilement.
Une solution mère de coelentéramine à 1,2 mM dans l'éthanol est préparée extemporanément et ajoutée aux cellules à la concentration finale de 2 µM.
La zéaxanthine est dissoute dans du DMSO stérile à la concentration de 1,2 mM et ajoutée aux cellules à la concentration de 2 µM.
Une solution stock de 10 mM d'ebselen est préparée à partir de 2,74 g de produit repris dans 1 ml d'éthanol puis la solution est aliquotée et conservée à -20°C.
La solution mère de 100 mM de désipramine est préparée extemporanément: 30,3 mg sont repris dans 1 ml d'eau distillée puis la solution est filtrée stérilement.
Une solution mère de 1 mM de fumonisine B1 est préparée à partir de 1 mg repris dans 1378 µ1 d'eau distillée puis la solution est stérilisée, aliquotée, congelée à -180°C et conservée à -20°C.
La solution mère de 9,4 mM de D609 est préparée extemporanément: 1 mg de D609 est repris dans 400 µl d'eau distillée puis la solution est filtrée stérilement.
Une solution stock de 30 mM du peptide z-VAD-FMK, z-DEVD- fmk, z-AEVD- fmk, z-VDVAD- fmk, z-IETD- fmk et z-LEHD-fmk est préparée à partir de 1 mg de produit stérile repris dans respectivement 70, 50, 54, 45, 51 et 42 µl de DMSO stérile. La solution de chaque inhibiteur est aliquotée en volumes adéquats, puis congelée à -20°C.

### 2) Appareils.

Les dosages spectrophotométriques ont été réalisés grâce au lecteur de plaque 96 puits iEMS Reader MF (Labsystems). Le fluorimètre (type LS 50B) provient de Perkin-Elmer. L'appareil à sonication (modèle B 15) provient de Branson. La détection de l'apoptose a été réalisée sous un microscope à fluorescence Axioplan de Carl Zeiss avec un filtre Zeiss IV, n° 9 (excitation: 450-490 nm ; émission >520 nm).

### II - Méthodes.

### 1) Culture des cellules microvasculaires rétiniennes bovines (Lecomte et al, 1996).

Les yeux de boeufs, fraîchement énucléés, sont transportés de l'abattoir sur de la glace, immédiatement débarrassés des tissus extraoculaires et placés dans du tampon PBS. Toutes les manipulations sont ensuite effectuées de façon stérile, sous hotte à flux laminaire. Deux yeux sont nécessaires pour chaque boîte de cellules à ensemencer. Les yeux sont plongés brièvement dans de l'éthanol à 70% puis rincés dans du milieu HBSS (sans Ca²⁺ ni Mg²⁺). Ils sont disséqués 5 mm postérieur à la cornée, la chambre antérieure de l'oeil avec le cristallin, ainsi que le vitré contenu dans le globe oculaire, sont enlevés. La rétine neuronale est décollée du globe oculaire, coupée au niveau du nerf optique et placée dans du tampon HBSS à 4°C. Ce tampon est préparé à partir de HBSS sans Mg²⁺ ni Ca²⁺ contenant 10 mM d'HEPES à pH 7,4, 100 U/ml de pénicilline et 100 µg/ml de streptomycine. Après 10 min d'oxygénation (95% d'O₂; 5% de CO₂), de la BSA à 22% est ajoutée jusqu'à une concentration finale de 0,5%; le pH est alors ajusté à 7,4 avec quelques gouttes de NaOH (en suivant le virage de l'indicateur rouge phénol). Les cellules pigmentaires contaminantes sont éliminées et les rétines, par lot de deux, sont placées dans 10 ml de tampon HBSS propre sur de la glace. Les rétines sont émincées avec des ciseaux (morceaux d'environ 2 mm), homogénéisées (Dounce 15 ml, piston A) avant d'être centrifugées (300 g, 5 min, 4°C).

### Culture des péricytes (BRP):

Deux rétines de boeuf sont utilisées pour chaque boîte (6 cm de diamètre) à ensemencer. Chaque lot de deux rétines est traité et homogénéisé comme décrit précédemment dans 10 ml de tampon HBSS à 4°C. L'homogénat est centrifugé (300 g, 5 min, 4°C) et le culot est resuspendu dans 5 ml d'une solution enzymatique de collagénase/dispase. Cette solution de collagénase/dispase (1 mg/ml) préparée dans du milieu HBSS sans Mg²⁺ ni Ca²⁺ contenant 10 mM d'HEPES à pH 7,4, 100 U/ml de pénicilline et 100 µg/ml de streptomycine, est ensuite oxygénée pendant 10 min et le pH est ajusté à 7,4 avant addition de TLCK (147 ng/ml) et de DNase (200 U/ml). La digestion est poursuivie pendant 20 min à 37°C et le culot cellulaire est resuspendu à la main à la mi-incubation. La suspension est alors filtrée sur un filtre nylon de mailles de 70 µm, stérile. Les fragments de microvaisseaux contenus sur le filtre sont récupérés dans du tampon HBSS (le filtre est rincé deux fois), centrifugés (300 g, 5 min, 4°C) et lavés une fois dans le même tampon. Après centrifugation (300 g, 5 min, 4°C), les fragments de microvaisseaux sont resuspendus dans 3 ml de DMEM contenant 10% de SVF, 2 mM de glutamine et les antibiotiques. Les fragments de microvaisseaux sont ensemencés dans une boîte de Pétri (6 cm de diamètre) enduite avec 100 µg de fibronectine (4 µg/cm²) dans 2 ml de DMEM pendant 3-4 heures à 37°C (Su & Gillies, 1992). Après avoir laissé les microvaisseaux s'attacher pendant 3 heures à 37°C, le milieu et les débris qui n'ont pas adhéré à la matrice sont éliminés et 3 ml du milieu de culture sont ajoutés. Ce milieu est constitué par du DMEM avec 10% de sérum de veau foetal (SVF), 80 µg/ml d'héparine, la glutamine et les antibiotiques. Les cellules sont maintenues dans un incubateur à 37°C, sous une atmosphère d'air humidifiée contenant 5% de CO₂. Le milieu de culture est renouvelé au bout de 24 heures puis toutes les 48 heures jusqu'à ce qu'elles atteignent la confluence.

A confluence, le milieu de culture est éliminé et les cellules sont trypsinées avec 1 ml d'une solution à 0,5% de trypsine et 0,2% d'EDTA jusqu'à ce que les cellules s'arrondissent (environ 5 minutes) après examen au microscope à contraste de phase. La trypsine est alors inactivée avec 2 ml de milieu de culture contenant du sérum et les cellules sont récupérées puis centrifugées (180 g, 3 min, 4°C). Les cellules, sous-cultivées dans un rapport 1:3, sont ensemencées dans trois boîtes (6 cm de diamètre) recouvertes de fibronectine et cultivées dans le même milieu.

### 2) Culture des péricytes en présence de produits avancés de glycation (AGE).

Les effets des AGE sont testés sur un même lot de cellules (BRP issus de la même culture primaire). Lorsqu'ils sont à confluence, les BRP en culture primaire sont trypsinés. Après les avoir laissés s'attacher 4 heures à 37°C, le milieu et les débris cellulaires qui n'ont pas adhéré à la matrice sont éliminés et le milieu de culture (10% de SVF pour les BRP) est ajouté. Afin de tester les effets des AGE, une solution d'AGE (ou son contrôle) est ajoutée au milieu de culture jusqu'à une concentration de 20 µM. Les cultures sont ainsi poursuivies pendant 1 ou 2 passages avec les agents, soit 7 et 15 jours respectivement pour les BRP.

### Préparation des AGE:

Des "AGE-méthylglyoxal" ont été préparés en incubant 7,2 mg/ml de BSA et 100 mM de méthylglyoxal pendant 50 heures à 37°C (Westwood & Thornalley, 1995). La BSA-contrôle a été obtenue en incubant de l'albumine dans les mêmes conditions mais sans méthylglyoxal. Les protéines glyquées ont été caractérisées par leur fluorescence (370-440 nm), leur fixation à des cellules endothéliales en culture, des phagocytes mononucléaires et le RAGE purifié (Yan *et al.,* 1994).

Les différentes préparations ont ensuite été dessalées sur des colonnes PD-10 (2,5 ml des préparations sont filtrés et récupérés dans 3,5 ml de DMEM).

### 3) Dosage des protéines par coloration au noir amide sur membrane de nitrocellulose.

Les protéines sont dosées selon la méthode décrite par Schaffner & Weissmann (1973). Les échantillons à doser contenant les protéines sont complétés à 270 µl avec du PBS avant d'ajouter 30 µl de tampon Tris-HCl (1 M, pH 7,5) contenant 1% de SDS, puis 60 µl d'acide trichloroacétique à 60%. Ces solutions sont "vortexées", laissées 2 min à température ambiante pour la précipitation des protéines et filtrées chacune au niveau d'un repère préalablement noté, sur un filtre de nitrocellulose de 0,45 µm, mouillé au TCA 6%. Une gamme étalon avec 0 à 27 µg de BSA est réalisée parallèlement. Tous les filtres sont ensuite immergés quelques minutes dans une solution de noir amide à 0,1% (w/v, dans méthanol: acide acétique: eau, 45:10:45, v/v/v). Les filtres sont alors rincés dans un bain d'eau puis dans la solution de décoloration (méthanol: acide acétique: eau, 90 :2 :8, v/v/v) jusqu'à ce que la membrane soit complètement blanche excepté au niveau des dépôts de protéines. Le filtre est séché, les dépôts sont découpés et le colorant fixé sur les protéines est élué par 1 ml de la solution d'élution (éthanol: eau: NaOH: EDTA, 100 :100 :0,1 :1,9, v/v/w/w) (environ 10 min en "vortexant" régulièrement). La lecture se fait sur 300 µl de ces solutions (plaque 96 puits) à 620 nm et la quantification est réalisée grâce au logiciel Biolise.

### 4) Evaluation immunocytochimique de l'apoptose dans les péricytes cultivés en présence d'AGE.

L'apoptose est détectée et quantifiée dans les péricytes cultivés pendant deux passages avec 2 µM d'AGE-méthylglyoxal ou de son contrôle en utilisant la technique de détection des cellules apoptotiques par l'annexine V et nécrotiques par l'iodure de propidium.

### Coloration à l'annexine V:

La présence de phosphatidylsérine (PS) sur le feuillet externe de la membrane plasmique, caractéristique des cellules apoptotiques, peut être détectée grâce à l'annexine-V marquée à la fluorescéine. Cette protéine a en effet une très forte affinité pour la PS. Afin de distinguer les cellules apoptotiques des éventuelles cellules nécrotiques qui fixent également l'annexine-V, cette coloration est effectuée en présence d'un agent intercalant et colorant de l'ADN, l'iodure de propidium, similaire au bromure d'éthydium. Pour ce marquage, les péricytes doivent être sous-confluents (75% de confluence) et n'ont donc été cultivés que 12 jours environ avec les AGE-méthylglyoxal ou BSA-contrôle.

Les BRP (boîte de 3,5 cm de diamètre) sont rincés trois fois avec du PBS puis incubés pendant 10 min dans le noir avec 500 µl d'un tampon HEPES contenant 10 µl de la solution d'annexine-V marquée à la fluorescéine et 10 µl de la solution d'iodure de propidium fournies dans la trousse de dosage. Les cellules apoptotiques ainsi que les cellules nécrotiques ont fixé l'annexine-V et apparaissent donc vertes sous le microscope à fluorescence, mais seul le noyau des cellules nécrotiques est coloré en rouge par l'iodure de propidium. Le pourcentage de cellules nécrotiques et de cellules apoptotiques est déterminé sur 10 champs différents, soit sur 300 à 800 cellules au total.

### 5) Dosage des céramides et diacylglycérols (DAG) .

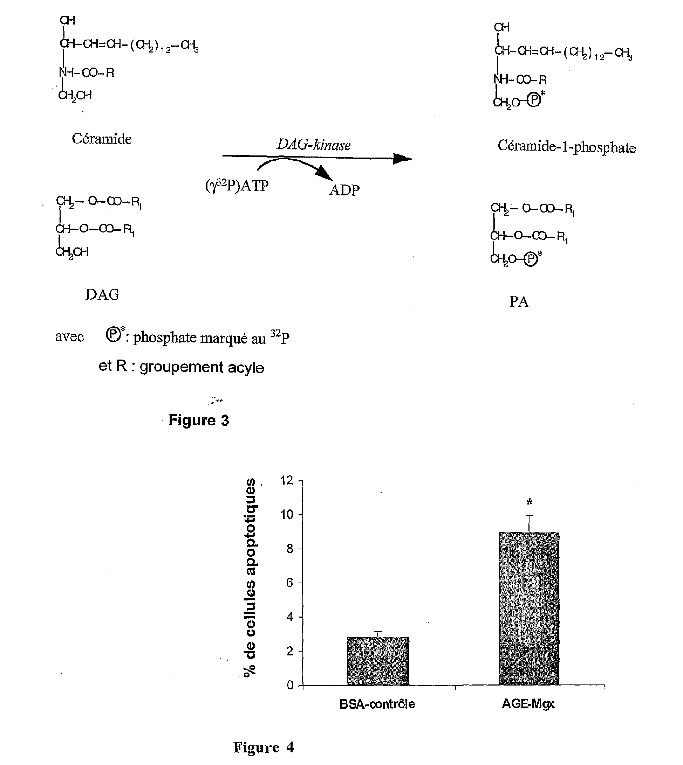

Les céramides et les diacylglycérols sont mesurés, dans les péricytes cultivés pendant deux passages avec 2 µM d'AGE-méthylglyoxal ou leur contrôle, selon la méthode décrite par Preiss et al. (1986). Cette méthode est basée sur la phosphorylation des céramides et des DAG par la diacylglycérol kinase (DAG-kinase) purifiée à partir d'Echerischia coli en présence d'ATP marqué au ³²P sur le phosphore y, selon le schéma représenté à la figure 3 en annexe.

Les péricytes (2 ou 3 boîtes de 6 cm de diamètre) sont rincés, récupérés par grattage au « rubber policeman » dans 0,5 ml d'eau sur glace et la boîte rincée par 0,5 ml supplémentaire (volume de 1 ml de suspension cellulaire) auquel sont ajoutés 10 µl d'une solution de [9, 10-³H] -triacylglycérol à 0,5 µCi/ml.

### 5.1) Extraction des lipides totaux.

Les lipides totaux sont ensuite extraits par la méthode de Bligh and Dyer (1959). A cette suspension cellulaire (1 ml de PBS) sont ajoutés 3.75 ml d'un mélange chloroforme: méthanol (2:1, v/v) contenant 80 µl de BHT (5 mM) en solution dans l'éthanol. Les solutions sont ensuite "vortexées" 30 sec et maintenues au repos 30 min minimum, puis 1,25 ml de NaCl (0.9%) et 1,25 ml de chloroforme sont ajoutés pour séparer les phases. Après centrifugation (700 g, 5 min), la phase organique inférieure est prélevée et l'extraction de la phase aqueuse est répétée deux fois avec 1,5 ml de chloroforme. Les extraits lipidiques sont alors rassemblés et le chloroforme est évaporé sous courant d'azote. La phase aqueuse permet de doser les protéines totales par coloration au noir amide et la phase organique, est récupérée dans un tube Eppendorff puis évaporée sous azote.

### 5.2) Phosphorylation à la DAG kinase.

Une gamme étalon de céramides (25 à 2500 ng) et de DAG (25 à 25000 ng) contenant la même quantité de triacylglycérol marqué au tritium est réalisée en parallèle. Les lipides sont ensuite solubilisés dans 20 µl d'une solution de détergents (7,5% de n-octylglucopyranoside, 5 mM de cardiolipine et 1 mM de DETAPAC) par 1 min de "sonication" suivie d'une incubation de 15 min à température ambiante. La réaction est démarrée par l'ajout de 50 µl du tampon de réaction (imidazole 100 mM, NaCl 100 mM, MgCl₂ 25 mM, EGTA 2 mM, pH 6,6), 20 µl d'une solution de DAG-kinase (4,86 mU) contenant 10 mM de DTT, et 10 µl de [γ-³²P] ATP à 5 mCi/ml dilué 10 fois dans une solution contenant 10 mM d'ATP non marqué, 100 mM d'imidazole et 1 mM DETAPAC (pH 6,6). La réaction est poursuivie pendant 1 heure à température ambiante puis arrêtée par 1 ml du mélange chloroforme: méthanol: acide chlorhydrique 1N (100 :100 :1, v/v/v), 170 µl d'un tampon contenant 10 mM d'HEPES, 135 mM de NaCl, 1,5 mM de CaCl₂, 0,5 mM de MgCl₂ et 5,6 mM de glucose (pH 7,4) puis 30 µl d'EDTA 100 mM. Le mélange est alors "vortexé", laissé 15 min à température ambiante puis centrifugé (10 000 g, 5 min) à température ambiante.

La phase organique (inférieure) est prélevée et évaporée sous azote. Les lipides sont alors repris par 300 µl du mélange éther: méthanol (9:1, v/v) et déposés sur une plaque de silice. Les céramides-1-phosphate sont séparés de l'acide phosphatidique et des triacylglycérols en utilisant le mélange chloroforme: méthanol: acide acétique (65 :15 :5, v/v/v) comme solvant de migration.

L'exposition de la plaque de silice à un film autoradiographique (30 min, -80°C) permet de visualiser les différents lipides marqués au ³²P. Les taches correspondant aux céramides-1-phosphate et à l'acide phosphatidique ainsi que les 3 derniers cm avant le front de migration (correspondant aux triacylglycérols) sont délimités au crayon à mine graphite et grattés. La silice est récupérée dans des pots de comptage, humidifiée par 200 µl de méthanol, 200 µl d'eau distillée et 2 ml de liquide de scintillation (Picofluor) pendant 2 heures avant de rajouter 8 ml du liquide de scintillation. La radioactivité émise par le ³²P ou le ³H est ensuite comptée dans chaque pot. La radioactivité émise par la solution mère de [9,10-³H]-triacylglycérol est également comptée, ce qui permet de calculer le rendement de la réaction pour chaque échantillon. Les quantités de céramides et diacylglycérols présentes dans chaque échantillon sont alors déterminées après extrapolation à partir de la gamme-étalon, en tenant compte du rendement, et rapportées à la quantité de protéines totales.

### PARTIE B: RÉSULTATS.

### I - EFFETS DES AGE-METHYLGLYOXAL SUR LES PERICYTES RETINIENS DE BOEUF.

Lors de travaux antérieurs in vitro, l'effet toxique d'AGE-glucose sur la prolifération des péricytes rétiniens a été mise en évidence (Ruggiero-Lopez et al, 1997).

L'étude s'est plus particulièrement intéressée aux effets des AGE-méthylglyoxal sur les péricytes rétiniens. Le méthylglyoxal semble en effet prendre toute son importance dans le diabète. Il s'agit d'un dicarbonyle dont la concentration sanguine est fortement augmentée chez le diabétique (McLellan et al, 1994). De plus, les produits avancés de glycation dérivant du méthylglyoxal ont été décrits comme les modifications chimiques majeures observées au cours de maladies chroniques comme le diabète (Degenhardt et al, 1998).

Les BRP ont été cultivés pendant deux passages (environ 15 jours) en présence des AGE-méthylglyoxal afin de mimer la chronicité d'un environnement diabétique, l'objectif de l'étude étant dans un premier temps de tester si les AGE-méthylglyoxal induisent l'apoptose des péricytes et si ce phénomène est associé à une production accrue de céramides et de DAG.

### 1) Apoptose des péricytes en réponse aux AGE-méthylglyoxal.

L'apoptose a été détectée et quantifiée par un marquage à l'annexine V et à l'iodure de propidium dans les BRP cultivés pendant deux passages avec 2 µM d'AGE-méthylglyoxal ou leur contrôle (voir Matériel et Méthodes). Cette technique met en évidence la translocation de la phosphatidylsérine du feuillet interne vers le feuillet externe de la membrane plasmique, phénomène biochimique caractéristique de la phase précoce de l'apoptose. L'annexine V, marquée à la fluorescéine, colore en vert les membranes plasmiques des cellules apoptotiques et nécrotiques mais seules les cellules nécrotiques, dont la membrane plasmique a perdu son intégrité, sont perméables à l'iodure de propidium qui colore leur noyau en rouge. La figure 4 rapporte l'effet des AGE-méthylglyoxal sur l'apoptose des péricytes rétiniens de boeuf. Les cellules ont été cultivées pendant deux passages (15 jours environ) en présence de 2 µM d'AGE-méthylglyoxal ou de BSA-contrôle. Les résultats sont exprimés en % de cellules apoptotiques dans la population cellulaire totale.

La proportion des cellules apoptotiques représente 8,9 ± 1,1% (moyenne ± SEM, n=10, p<0,05) du total des péricytes cultivés avec 2 µM d'AGE-méthylglyoxal contre seulement 2,9 ± 0,34% des cellules cultivées avec 2 µM BSA-contrôle. Ainsi les AGE-méthylglyoxal induisent une augmentation environ d'un facteur 3 en moyenne du taux d'apoptose dans les péricytes. Aucune cellule nécrotique n'a été observée.

### 2) Production de céramides et de DAG dans les péricytes.

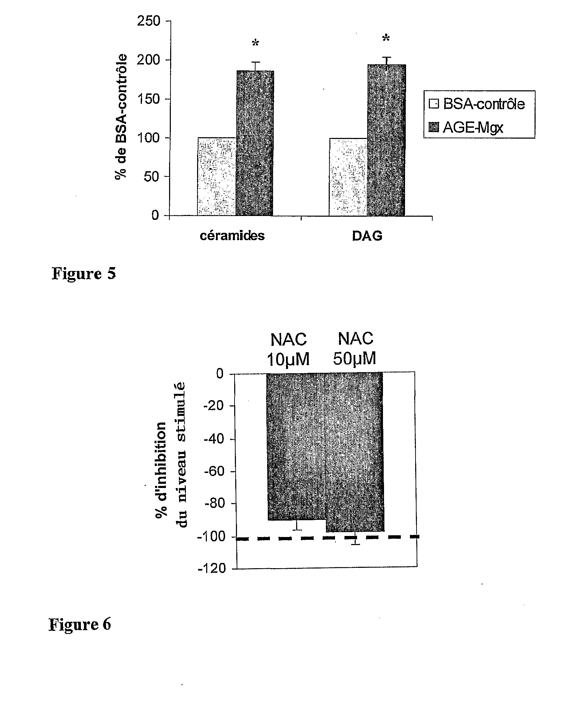

Afin de déterminer si les céramides et les DAG pouvaient être impliqués dans le signal de transduction menant à l'apoptose, ces deux composés ont été dosés dans les péricytes incubés pendant deux passages (15 jours environ) avec 2 µM d'AGE-méthylglyoxal ou leur contrôle. La technique de dosage employée est basée sur la phosphorylation des céramides et des DAG par la DAG-kinase en présence d'ATP radioactif (³²P) (voir Matériel et Méthodes). La figure 5 rapporte l'effet des AGE-méthylglyoxal sur la production de céramides et de DAG dans les péricytes rétiniens de boeuf. Les résultats sont exprimés en % de variation du contrôle cellules cultivées avec BSA contrôle.

Une stimulation de la production des céramides (+ 85 ± 12%) (moyenne ± SEM, n=10, p<0,05) et des DAG (+ 94 ± 9%) a pu être mise en évidence dans les BRP en réponse aux AGE-méthylglyoxal.

### 3) Conclusion.

Un marquage à l'annexine V et à l'iodure de propidium des cellules cultivées pendant 15 jours avec 2 µM d'AGE-méthylglyoxal a permis de détecter une augmentation du taux d'apoptose des BRP d'un facteur 3, en moyenne. Ceci suggère que les AGE-méthylglyoxal induisent à long terme l'apoptose des péricytes en culture. La disparition des péricytes rétiniens par apoptose a été décrite *in vitro* en réponse à des fluctuations abruptes de glucose (Li *et al.,* 1996) et *ex vivo* dans la rétine de patients et de rats diabétiques (Mizutani et al., 1996; Li *et al.*, 1997). L'altération de l'expression de gènes régulateurs de l'apoptose (ARNm de la protéine Bax) a également été mise en évidence dans les péricytes cultivés à long terme avec de fortes concentrations de glucose puis soumis à une réduction rapide en glucose (Li *et al.*, 1998) et dans les péricytes de rétines de patients diabétiques récupérées post mortem (Podesta *et al*., 2000).

En revanche, l'induction de l'apoptose des péricytes rétiniens par des AGE (les AGE-glucose et AGE-méthylglyoxal) n'a jamais été mise en évidence précédemment.

Les travaux réalisés dans le cadre de l'invention ont permis de montrer que l'apoptose des péricytes induite par les AGE-méthylglyoxal est associée à une stimulation de la production de céramides et de DAG, démontrant ainsi que ces deux composés sont impliqués dans la transduction du signal apoptotique. Les travaux suivants ont consisté à décrire la voie de signalisation menant à l'apoptose des péricytes en réponse aux AGE-méthylglyoxal.

### II - INDUCTION D'UN STRESS OXYDANT PAR LES AGE-METHYLGLYOXAL DANS LES PERICYTES RETINIENS EN CULTURE.

Des travaux antérieurs ont permis de démontrer une atteinte des capacités antioxydantes des péricytes rétiniens de boeuf, en réponse aux AGE-Glucose (20 µM). Une augmentation des activités de la catalase et de la Cu/Zn SOD ainsi que la quantité de thiols réduits a été observée dans les péricytes cultivés de façon chronique avec les AGE-Glucose, suggérant une réponse compensatoire des cellules face à une production accrue de radicaux libres et donc à un stress oxydant (Paget et al, 1998). Une diminution des marqueurs de la peroxydation lipidique, les TBARS et les isoprostanes, a également été observée comme le reflet en miroir d'une stimulation des défenses antioxydantes (Paget et al, 1998).

Afin de vérifier l'implication d'un stress oxydant dans la voie de signalisation menant à l'apoptose, les effets de différentes classes d'antioxydants sur l'apoptose induite des péricytes et sur la production stimulée des céramides et des DAG en réponse aux AGE-méthylglyoxal ont été étudiés.

### 1) Les différents antioxydants testés.

Sur la base de leur structure moléculaire et de leur mode d'action, les antioxydants testés peuvent être classés en 3 groupes. Le premier groupe comprend des composés hydrosolubles possédant une fonction thiol qui a la capacité de fournir un proton: il s'agit de la Nα-acétyl-L-Cystéine (NAC) et de l'acide lipoïque. Ces deux agents présentent un effet protecteur de la balance rédox du cytoplasme de la cellule. Le deuxième type d'antioxydant testé est l'ebselen, une molécule de synthèse contenant un atome de sélénium. Ce composé présente la particularité de mimer l'activité glutathion-peroxydase sélénium dépendante (Se-GPx). Il induit ainsi une protection des membranes contre la peroxydation lipidique. Le 3^{ème} groupe est constitué de composés liposolubles riches en doubles liaisons conjuguées qui présentent une haute réactivité vis-à-vis des radicaux libres et qui protègent ainsi plus spécifiquement les membranes contre la peroxydation lipidique. Il s'agit de la zéaxanthine et de la coelentéramine.

### 2) Effets de la N-acétyl-L-Cystéine.

La figure 6 rapporte l'effet de la Nα-acétyl-L-Cystéine sur l'apoptose (annexine V, voir Matériel et Méthodes) induite des BRP par les AGE-méthylglyoxal. Les péricytes ont été cultivés pendant deux passages (de 12 à 15 jours) en présence de 2 µM AGE-méthylglyoxal (ou de la BSA-contrôle) et de 10 µM ou 50 µM de N-acétyl-L-Cystéine. Les résultats, exprimés en % d'inhibition de l'apoptose induite, sont la moyenne de 5 expériences indépendantes.

Aux deux concentrations testées, la N-acétyl-L-Cystéine protège les péricytes de l'apoptose induite par les AGE-Métylglyoxal.

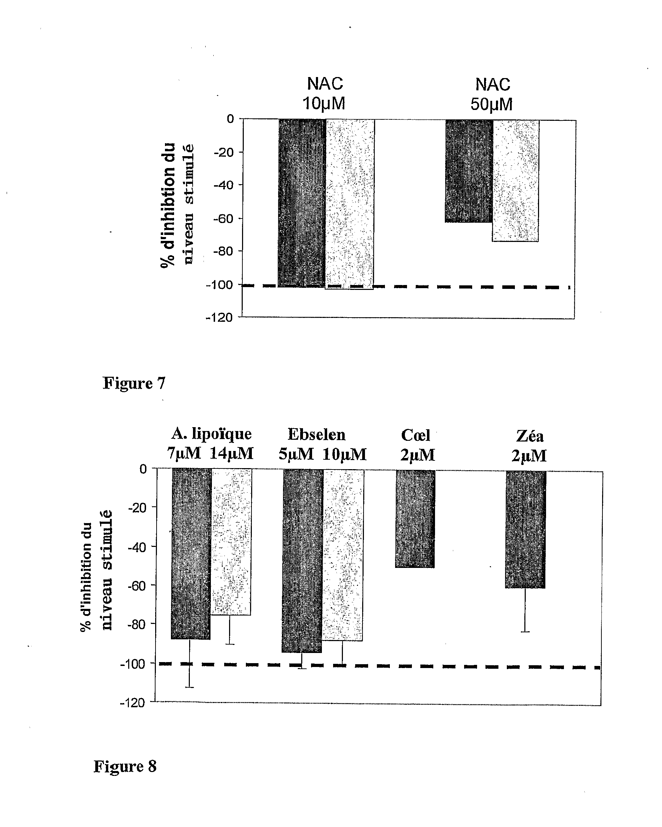

Les céramides et les DAG ont été également dosés dans les BRP cultivés en présence de N-acétyl-L-Cystéine (voir figure 7). Les céramides et les DAG ont été dosés comme décrit dans la section Matériel et Méthodes. Les résultats, exprimés en % d'inhibition de production stimulée de céramides et de DAG, sont la moyenne de 2 expériences indépendantes. L'ajout de 10 µM de N-acétyl-L-Cystéine dans le milieu de culture inhibe totalement la production de céramides et de DAG induite par les AGE-méthylglyoxal (-100%). En revanche, un traitement des cellules par 50 µM de N-acétyl-L-Cystéine n'induit qu'une inhibition partielle de cette surproduction (-60% de la surproduction des céramides et -70% de la surproduction de DAG). Cet effet partiel peut probablement être expliqué par un effet prooxydant d'un antioxydant employé à une concentration trop élevée.

L'inhibition complète de l'apoptose induite des péricytes cultivés en présence de 10 µM de N-acétyl-L-Cystéine a été associée à une inhibition parallèle totale de la surproduction des céramides et des DAG, ceci suggère donc que les AGE-méthylglyoxal induisent un stress oxydant situé en amont de la production de céramides et des DAG ainsi que de l'apoptose des péricytes.

### 3) Effets de divers antioxydants sur l'apoptose des péricytes en réponse aux AGE-méthylglyoxal.

L'effet protecteur de la Nα-acétyl-L-Cystéine sur la disparition des péricytes en réponse aux AGE-méthylglyoxal a été confirmé en testant différents antioxydants. La figure 8 rapporte les effets des antioxydants sur l'apoptose des péricytes en réponse aux AGE-méthylglyoxal. Les résultats, exprimés en % d'inhibition de l'apoptose induite, sont la moyenne de 3 expériences indépendantes sauf pour la coelentéramine (2 expériences indépendantes).

L'ajout dans le milieu de culture d'acide lipoïque aux concentrations de 7 µM et de 14 µM inhibe respectivement de 88% et de 75% l'apoptose (annexine V, voir Matériel et Méthodes) des péricytes induite par les AGE. Une incubation des cellules avec 5 µM et 10 µM d'ebselen bloque respectivement à 94% et 88% l'apoptose induite par les AGE-méthylglyoxal. En revanche, un traitement des péricytes avec 2 µM de coelentéramine et 2 µM de zéaxanthine ne permet qu'une inhibition partielle de l'apoptose induite de 60% et de 50 % respectivement. Cet effet modéré des deux antioxydants liposolubles peut être expliqué par le fait que ces deux agents n'ont pas pu être testés à des concentrations plus élevées: un effet véhicule (le DMSO) a été observé sur l'apoptose des péricytes en réponse aux AGE-méthylglyoxal.

Une incubation des péricytes avec différents antioxydants permet de protéger les cellules de l'apoptose. Ce résultat confirme bien l'induction d'un stress oxydant dans la voie de signalisation de l'apoptose des péricytes induite par les AGE-méthylglyoxal.

### III - VOIES DE PRODUCTION DES CERAMIDES ET DES DAG ACTIVEES DANS LES PERICYTES RETINIENS EN REPONSE AUX AGE-METHYLGLYOXAL.

Les travaux réalisés dans le cadre de l'invention ont également consisté à définir les voies métaboliques de production des céramides et des DAG activées dans les péricytes cultivés avec des AGE-méthylglyoxal.

Il s'agissait notamment de déterminer les différentes voies possibles de production des céramides. D'une part, les céramides peuvent être issus de la voie de biosynthèse de *novo*. Deux intermédiaires métaboliques, la sphingosine et la sphinganine sont acétylés par une sphinganine-acyl-transférase pour former des céramides.

Les céramides peuvent être également le produit d'hydrolyse enzymatique : ils peuvent être issus d'une part du « pool » de sphingomyélines et d'autre part du « pool » de glycosphingolipides (céramides liés à une ou plusieurs chaînes glycanniques) (Mathias et al, 1998; Okasaki et al., 1998).

De même, les voies de production des DAG sont de deux types. Ils peuvent provenir d'une activation de la voie de biosynthèse *de novo,* ou alors être issus d'hydrolyses enzymatiques: les DAG peuvent être libérés à partir d'un « pool » de phosphatidylinositolbisphosphates ou d'un « pool » de phosphatidylcholines sous l'action de phospholipases spécifiques.

Les voies de production de ces deux intermédiaires métaboliques ont été explorées selon une approche pharmacologique. Les cellules ont été cultivées en présence de différents inhibiteurs enzymatiques, dont les effets ont été analysés d'une part sur l'apoptose induite et d'autre part sur la production stimulée de céramides et de DAG en réponse aux AGE-méthylglyoxal.

### 1) La Voie de production des céramides activée dans les péricytes.

### a) Implication d'une sphinganine-acyl-transférase.

Afin de vérifier si la voie de biosynthèse *de novo* est activée en réponse aux AGE-méthylglyoxal, l'effet d'un inhibiteur spécifique de la sphinganine-acyl-transférase sur l'apoptose des péricytes, la fumonisine B1 (Wang et al, 1991), a été testé.

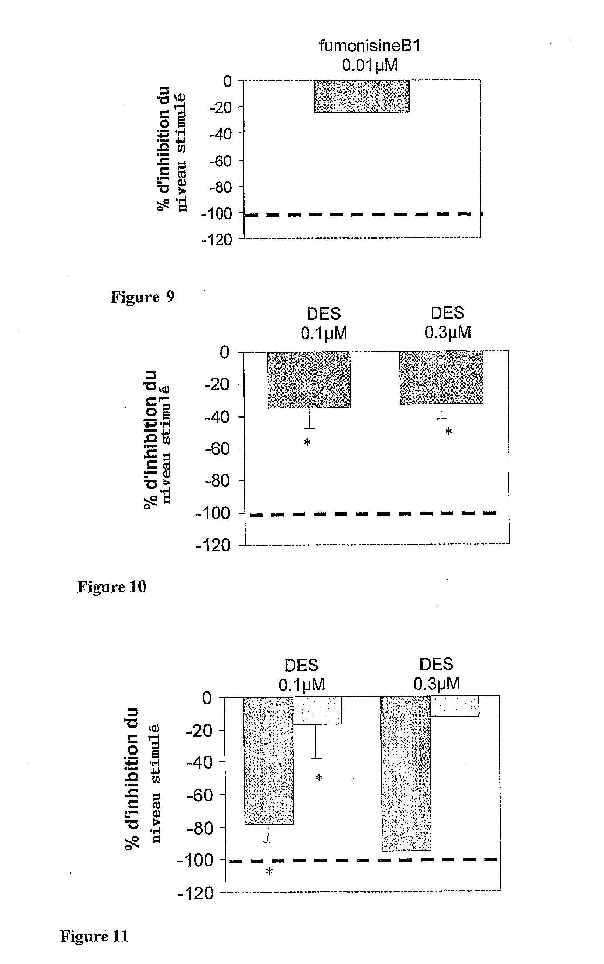

La figure 9 rapporte l'effet de la fumunisine B1 sur l'apoptose des péricytes induite par les AGE-méthylglyoxal. Les résultats, exprimés en % d'inhibition de l'apoptose induite, sont la moyenne de 2 expériences indépendantes. L'ajout de 0,01 µM de fumonisine B1 dans le milieu de culture n'a induit qu'une inhibition de 22% de l'apoptose des péricytes (annexine V, voir Matériel et Méthodes) cultivés pendant deux passages (15 jours environ) en présence d'AGE-méthylglyoxal (ou de BSA-contrôle). La fumonisine B1 à 0,1 µM a également été testée. A cette concentration, la fumonisine B1 est proapoptotique, puisqu'elle affecte le taux basal d'apoptose des cellules cultivées avec de la BSA-contrôle (résultats non montrés).

La fumonisine B1 ne protège pas les péricytes de l'apoptose induite. La synthèse *de novo* des céramides ne semble donc pas être impliquée dans l'apoptose des péricytes rétiniens induite par les AGE-méthylglyoxal.

### b) Implication d'une sphingomyélinase acide.

Les céramides peuvent être formés à partir du « pool » de sphingomyélines sous l'action de sphingomyélinases. Il existe différentes sphingomyélinases qui se distinguent par leur localisation intracellulaire et par leur pH optimal d'activité: il s'agit des sphingomyélinases acide, neutre et basique.

L'implication d'une sphingomyélinase acide a été vérifiée en analysant les effets d'un inhibiteur spécifique de cet enzyme, la désipramine (10,11-Dihydro-5-[3-(methylamino)propyl]-5H-dibenz [b,f] azepine) (Andrieu et al., 1994; Jaffrezou et al., 1995), sur l'apoptose induite des cellules et sur la production stimulée des céramides et des DAG dans les péricytes cultivés avec les AGE-méthylglyoxal.

La figure 10 rapporte l'effet de la désipramine sur l'apoptose des péricytes induite par les AGE-méthylglyoxal. Les résultats, exprimés en % d'inhibition de l'apoptose induite, sont la moyenne de 7 expériences indépendantes pour la désipramine à 0,1 µM et de 6 expériences indépendantes pour la désipramine à 0,3 µM. Une culture des péricytes pendant deux passages (15 jours environ) en présence d'AGE-méthylglyoxal avec 0,1 µM et 0,3 µM de désipramine permet une inhibition partielle de 35% de l'apoptose induite par les AGE-méthylglyoxal (Figure 10). De la désipramine à 1 µM a également été ajoutée dans le milieu de culture mais; dans ces conditions, le taux basal d'apoptose des cellules-contrôle a été affecté (résultats non montrés).

La figure 11 rapporte l'effet de la désipramine sur la production stimulée de céramides et de DAG dans les BRP induites par les AGE-méthylglyoxal. Les céramides et les DAG ont été dosés comme décrit dans la section Matériel et Méthodes. Les résultats, exprimés en % d'inhibition de production stimulée de céramides et de DAG, sont la moyenne de 4 expériences indépendantes pour la désipramine à 0,1 µM et de 2 expériences indépendantes pour la désipramine à 0,3 µM. La désipramine à 0,1 µM ajoutée dans le milieu de culture pendant deux passages bloque 78% des céramides produits dans les péricytes en réponse aux AGE-méthylglyoxal mais ne réduit que de 17% la surproduction de DAG. L'effet inhibiteur de la désipramine à 0,3 µM sur la production stimulée des céramides semble être dose-dépendant puisque l'agent testé induit une inhibition de 95% des céramides formés. La surproduction des DAG n'est bloquée que de 13%. L'ajout de 1 µM de désipramine dans le milieu de culture a affecté le taux basal des céramides et des DAG des cellules cultivées avec la BSA-contrôle (résultats non montrés).

### c) Conclusions.

Un traitement des péricytes avec 0,3 µM de désipramine induit une inhibition complète des céramides produits sans affecter la surproduction des DAG: l'augmentation observée des céramides dans les péricytes cultivés avec 2 µM d'AGE-méthylglyoxal est bien liée à l'activation d'une sphingomyélinase acide. Cet enzyme pourrait être couplé à une phospholipase C spécifique des phosphatidylcholines (PC-PLC) (Schütze et al, 1992; Genestier et al, 1998). Ainsi dans les péricytes cultivés avec 2 µM d'AGE-méthylglyoxal, l'action d'une PC-PLC sur un pool de phosphatidylcholines libérerait des DAG qui activeraient en cascade une sphingomyélinase acide induisant la formation de céramides. Lorsque les cellules sont cultivées avec de la désipramine, la sphingomyélinase acide est inhibée: il en résulte une baisse de la production des céramides alors que les DAG, produits en amont de la cible enzymatique, ne sont pas affectés.

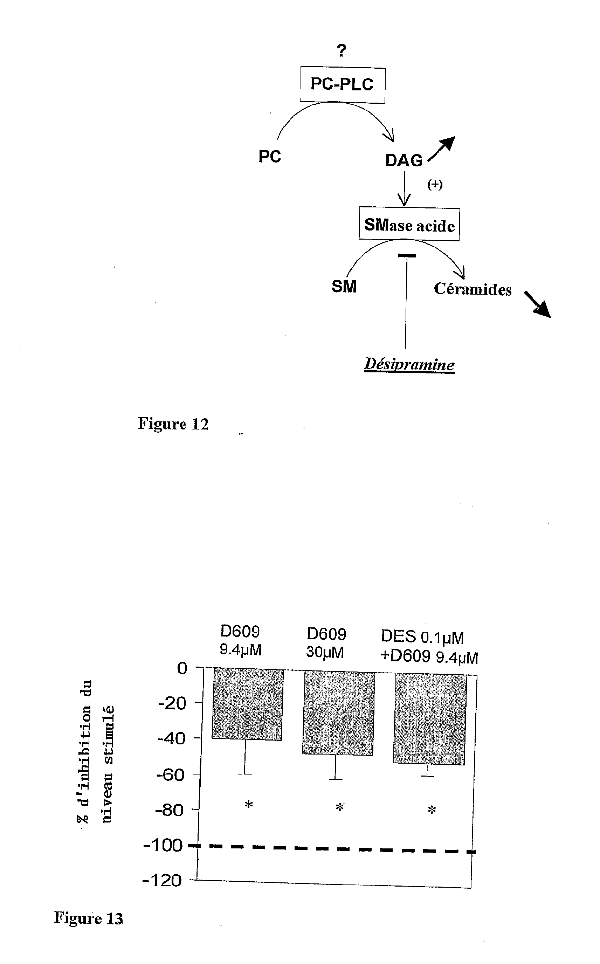

La figure 12 rapporte la voie de production des céramides et des DAG dans les péricytes envisagée sur la base de l'invention.

Bien que la surproduction des céramides ait été totalement bloquée, la désipramine ne protège que partiellement les péricytes de l'apoptose induite par les AGE-méthylglyoxal.

### 2) Voie de production des DAG activée dans les péricytes.

### a) Implication d'une phosphatidylcholine-phospholipase C.

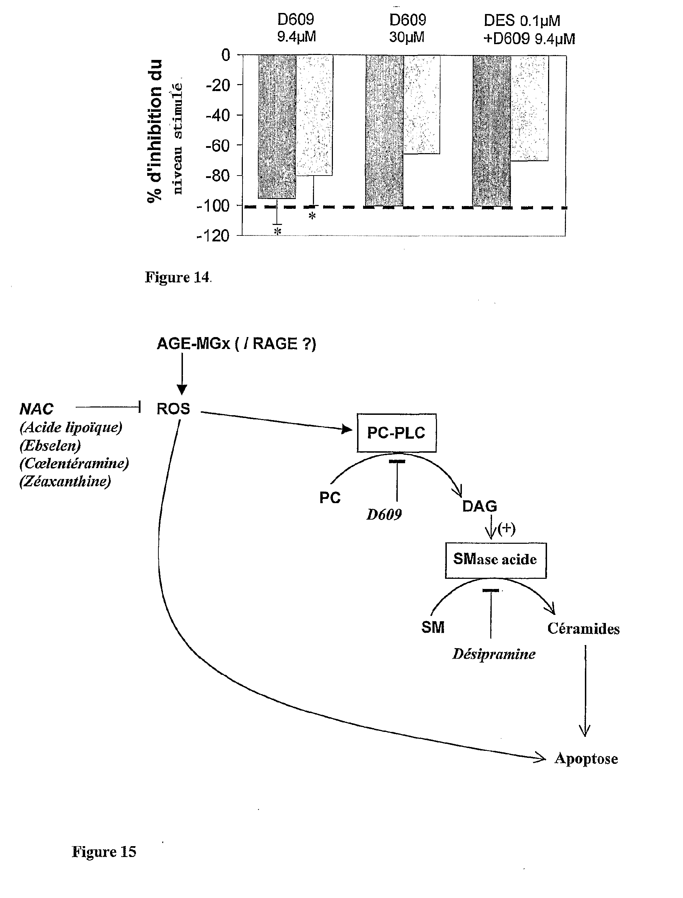

Afin de confirmer, d'une part, l'hypothèse proposée pour la voie de production des céramides et des DAG et de vérifier, d'autre part, si les DAG sont impliqués dans l'apoptose résiduelle, les effets d'un inhibiteur spécifique de la phosphatidylcholine-phospholipase C, le D609 (tricyclo[5.21.0(2.6)]decyl-9[8]xanthogenate) (Müller-Decker et al, 1988; Müller-Decker , 1989), sur l'apoptose induite (Figure 13) et sur la production des céramides et des DAG induite par les AGE-méthylglyoxal (Figure 14) a été analysée.

La figure 13 rapporte l'effet du D609 sur l'apoptose (annexine V, Matériel et Méthodes) des péricytes induite par les AGE-méthylglyoxal après deux passages. Les résultats, exprimés en % d'inhibition de l'apoptose induite, sont la moyenne de 7 expériences indépendantes pour le D609 à 9,4 µM et de 6 expériences indépendantes pour le D609 à 30 µM et la combinaison désipramine/D609. L'ajout de 9,4 µM et 30 µM de D609 dans le milieu de culture a permis de bloquer respectivement de 40% et 47% l'apoptose des péricytes induite par les AGE-méthylglyoxal. Nous avons également testé la combinaison 0,1 µM de désipramine/ 9,4 µM de D609, dans l'optique d'agir simultanément sur les deux cibles enzymatiques. Un traitement des cellules par les deux agents combinés induit un effet équivalent au D609 seul: l'apoptose des péricytes a été inhibée de 51%.

La figure 14 rapporte l'effet du D609 sur la production stimulée de céramides et de DAG (voir Matériel et Méthodes) dans les BRP induites par les AGE-méthylglyoxal. Les résultats, exprimés en % d'inhibition de la production stimulée de céramides et de DAG, sont la moyenne de 4 expériences indépendantes pour le D609 à 9,4 µM et de 2 expériences indépendantes pour le D609 à 30 µM et la combinaison désipramine/D609. Une incubation des cellules en présence de 9,4 µM de D609 induit une inhibition presque totale de la production des céramides et des DAG de 95% et de 80% du niveau stimulé, respectivement. A la concentration de 30 µM le D609 a permis une inhibition complète de la production des céramides et une inhibition de 65% des DAG produits. Un traitement des cellules par les deux agents combinés induit un effet équivalent au D609 30 µM: la surproduction des céramides est totalement bloquée et 70% des DAG produits sous AGE-méthylglyoxal sont inhibés.

### b) Conclusion.

Une incubation des péricytes avec du D609, plus particulièrement à la concentration de 9,4 µM, induit une inhibition parallèle de la production stimulée des céramides et des DAG. Ce résultat permet donc de conclure que la formation des DAG est bien liée à l'activation d'une phosphatidylcholine-phospholipase C couplée à une sphingomyélinase acide, ce qui valide la voie de production des céramides et des DAG envisagée précédemment.

Toutefois, malgré une inhibition complète des céramides et des DAG, les cellules ne sont que partiellement protégées de l'apoptose induite par les AGE-méthylglyoxal, ceci suggère l'existence d'une seconde voie de signalisation parallèle à la production des céramides et des DAG et menant à l'apoptose des péricytes.

La figure 15 montre la voie de signalisation menant à l'apoptose des péricytes.

Les AGE-méthylglyoxal, probablement par l'intermédiaire d'un récepteur spécifique aux AGE, induisent un stress oxydant qui peut être situé au carrefour de deux voies de transduction de signal menant à l'apoptose des péricytes. Effectivement, un traitement des cellules avec de la N-acétyl-L-Cystéine bloque complètement la production stimulée des céramides et des DAG et inhibe totalement l'apoptose des péricytes induite par les AGE-méthylglyoxal.

Les travaux de recherche ont alors consisté à définir une cible enzymatique située en amont des céramides et des DAG et qui pourrait également se trouver au carrefour des deux voies de signalisation.

### IV - IMPLICATION DE CASPASES: LES PROTEASES SPECIFIQUES DE L'APOPTOSE.

Des études récentes ont mis en évidence qu'une série de protéases de la famille des ICE (Interleukin-1 Converting Enzyme) renommées caspases (Cytosolic Asparte-Specific Cysteine Proteases) jouaient un rôle clef dans la transduction des signaux apoptotiques. En fonction du critère de comparaison, différentes classifications de ces protéases ont été proposées:
- Selon leur temps d'implication dans la voie de signalisation de l'apoptose, ces enzymes ont été classées en deux groupes: les caspases initiatrices, activées au cours la phase d'initiation de l'apoptose (caspases-2, 8, 10) et les caspases effectrices, pouvant être activées en cascade par des caspases initiatrices, impliquées dans la phase d'exécution de l'apoptose qui se caractérise par des atteintes biochimiques et morphologiques de la cellule (caspases-3, 6, 7, 9) (Nunez et al, 1998; Stennicke et al, 1998; Alnemri, 1999).
- En revanche, sur la base de leur spécificité de substrat, les caspases ont pu être classées différemment en trois sous groupes:, le groupe I constitué des caspases-1, 4, 5 (enzymes décrits comme étant impliqués dans les réponses inflammatoires), le groupe II constitué des caspases-2, 3, 7 et le groupe III constitué des caspases-6, 8, 9, 10. Alors que les protéases du groupe II exigent un résidu aspartate aussi bien en première qu'en quatrième position du site de clivage protéolytique (XDXXD), les protéases du groupe III sont plus tolérantes vis-à-vis du quatrième résidu aminoacide, tout en présentant une préférence pour les acides aminés aliphatiques (XE[I/T/H/V]D) (Thornberry et al, 1997; Garcia-Calvo et al, 1998).
- Enfin une analyse phylogénétique des caspases a permis de définir trois sous-familles: les ICE-like protéases (famille des caspases-1) regroupant les caspases-1, 4, 5, 11, les IchII-like protéases (famille des caspases-2) regroupant les caspases-2, 9 et les CPP (cysteine protein protease)32-like protéases (famille des caspases-3) regroupant les caspases-3, 6, 7, 8, 10 (Alnemri et al, 1996; Duan et al, 1996a, 1996b).

Les caspases sont inhibées de façon spécifique *in vitro* et *in vivo* par des peptides synthétiques (le plus souvent des tétrapeptides) rendus perméables aux cellules par des groupements chimiques bloquant les fonctions NH₂ et COOH terminales et mimant le site de clivage de leur substrat respectif.

Différents peptides inhibiteurs synthétiques ont été testés afin de vérifier si des caspases initiatrices et/ou effectrices étaient impliquées dans l'apoptose des péricytes induite par les AGE-méthylglyoxal.

Trois peptides décrits comme des inhibiteurs spécifiques d'une protéase ont été testés:
- le peptide z-VDVAD-fmk inhibiteur spécifique de la caspase-2 initiatrice,
- le peptide z-IETD-fmk inhibiteur spécifique de la caspase-8,
- le peptide z-LEHD-fmk inhibiteur spécifique de la caspase-9.

Nous avons également analysé les effets d'autres peptides modifiés ne présentant pas de spécificité vis-à-vis d'une caspase ou bien une spécificité modérée dépendant de l'accessibilité de l'enzyme et de la concentration intracellulaire de l'inhibiteur:
- l'inhibiteur z-VAD-fmk qui inhibe un large spectre de caspases, incluant ainsi les caspases initiatrices et les caspases effectrices (Garcia-Calvo et al, 1998);
- le peptide z-DEVD-fmk qui présente un effet inhibiteur des CPP32-like protéases, agissant principalement sur la caspase-3 effectrice (Nicholson et al, 1995) et de façon moins spécifique sur les caspases-6, 7, 8, 10 (Fernandes-Alnemri et al, 1995);
- le peptide z-AEVD-fmk qui est un inhibiteur de la caspase-10 initiatrice et de façon moins spécifique des caspases-8, 9.

### 2) Effet des peptides inhibiteurs de caspases sur l'apoptose induite des péricytes.

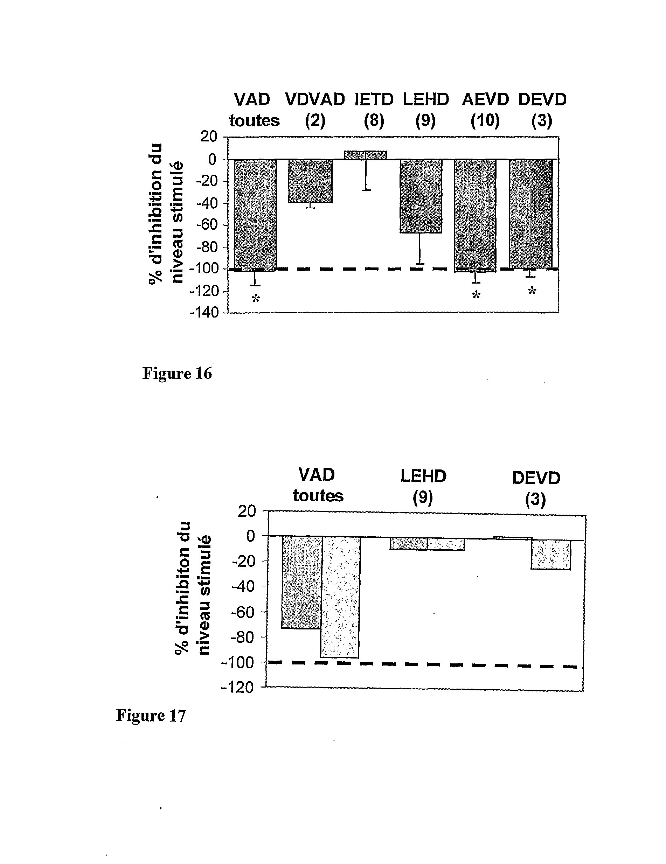

La figure 16 rapporte l'effet des peptides inhibiteurs de caspases sur l'apoptose des péricytes induite par les AGE-méthylglyoxal. Les cellules ont été cultivées pendant deux passages (15 jours environ) en présence d'AGE-méthylglyoxal (ou de BSA-contrôle) et des peptides inhibiteurs z-VAD-fmk, z-DEVD-fmk, z-IETD-fmk, z-AEVD-fmk, z-VDVAD-fmk et z-LEHD-fmk (50 µM). L'apoptose a été détectée et dosée par un marquage à l'annexine V, comme décrit dans la section Matériel et Méthodes. Les résultats exprimés en % d'inhibition de l'apoptose induite sont la moyenne de 6 expériences indépendantes pour z-VAD-fmk et z-DEVD-fmk, de 4 expériences indépendantes pour z-LEHD-fmk et de 3 expériences indépendantes pour z-VDVAD-fmk, z-AEVD-fmk et z-IETD-fmk.

L'ajout de 50 µM de z-VAD-fmk, z-DEVD-fmk, z-AEVD-fmk et z-LEHD-fmk dans le milieu de culture permet une inhibition quasi totale de l'apoptose des péricytes induite par les AGE-méthylglyoxal. Alors que le peptide z-IETD-fmk ne protège pas les cellules de l'apoptose, le peptide z-VDVAD-fmk induit une inhibition partielle de l'apoptose induite par les AGE-méthylglyoxal (Figure 16).

### 3) Effet des peptides inhibiteurs de caspases sur la production stimulée des céramides et des DAG dans les péricytes rétiniens.

La figure 17 montre l'effet des peptides inhibiteurs de caspases z-VAD-FMK et z-DEVD-FMK (50 µM) sur la production stimulée de céramides et de DAG dans les BRP induites par les AGE-méthylglyoxal. Les céramides et les DAG ont été dosés comme décrit dans la section Matériel et Méthodes. Les résultats sont exprimés en % d'inhibition de la production stimulée et sont le résultat de 2 expériences indépendantes.

Les effets des inhibiteurs peptidiques sur la production des céramides et des DAG diffèrent: alors que la production stimulée des deux intermédiaires métaboliques n'est pas affectée dans les péricytes traités avec 50 µM de z-DEVD-fmk ou de z-LEHD-fmk, le peptide z-VAD-fmk bloque la surproduction des céramides et des DAG de 75% et 100% respectivement.

### 4) Implication de la caspase 10 dans l'apotose des péricytes rétiniens provoquée par les AGE.

### a) Matériel.

L'anticorps primaire dirigé contre la caspase-10, son peptide bloquant respectif, la caspase-10 recombinante utilisée comme contrôle positif et l'anticorps secondaire anti-lapin ont été fournis par Biomol (France) Les films pour autoradiographie (Hyperfilm) et les réactifs de révélation ECL ont été achetés chez Amersham (France). Les gels de SDS-page (ready gel Tris-HCl 12%), les membranes de nitrocellulose (porosité de 0,45 µM) pour les immunoempreintes, l'anticorps secondaire anti-chèvre et les marqueurs de poids moléculaires (protein precision standards, broad range) sont de Bio-Rad (France).

Pour l'électrophorèse et l'électrotransfert des protéines, les systèmes Mini-Protean II et Mini-Trans Blot de Bio-Rad ont été utilisés.

### b) Immunoblot.

### - Préparation des échantillons

Les péricytes (2 à 3 boites de 6 cm), cultivés pendant 15 jours en présence de 3 µM d'AGE-méthyglyoxal (ou BSA-contrôle) comme décrit dans l'application sus-nommée, sont déposés sur un lit de glace, lavés 3 fois par du DPBS froid puis grattés à l'aide d'un ruber policeman dans 500 µl de tampon de récupération froid (Tris-HCl 0.1 M pH 8 contenant 4.4 µM de pepstatine A, 6 µM de leupeptine, 0.6 mM de PMSF et 10 mM d'EDTA). Afin de limiter la perte de matériel cytoplasmique, la suspension cellulaire est solubilisée directement dans la boite de pétri par addition de 100 µl de tampon Laëmmli 5X (Tris-HCl 0,15 M pH6,8 contenant 5% de SDS, 12.5 % de glycérol, 12.5% de βmercaptoéthanol et 1% de bleu de bromophénol). Les pelotes d'ADN, rendant la solution visqueuse, sont cassées mécaniquement à l'aide de va-et-vient énergiques dans une seringue équipée d'une aiguille 30G_{1/2}. Les échantillons protéiques sont alors chauffés 3 min à 100°C avant d'être déposés sur le gel de polyacrylamide.

### - Electrophorèse sur gel de polyacrylamide en condition dénaturante (SDS-page):

L'électrophorèse est réalisée dans des conditions réductrices (β-mercaptoéthanol) et dénaturantes (SDS) selon la technique de Laëmmli (Laëmmli U.K, 1970).

Des aliquotes de lysats de péricytes (20 µg) sont déposées sur un gel de polyacrylamide (ready gel Tris-HCl de Bio-Rad), constitué d'un gel de concentration de 4% d'acrylamide et d'un gel de séparation de 12% d'acrylamide. L'électrophorèse des protéines est effectuée à 100 V (gel de concentration) puis 200 V (gel de séparation) dans le tampon de migration (Tris 25 mM, glycine 0,19 M, SDS 0.1%), jusqu'à ce que le bleu de bromophénol (repérant le front de migration) atteigne le bas du gel. Des marqueurs de masse moléculaire (250, 150, 100, 75, 50, 37, 25, 15, 10 kDa) sont également déposés afin d'étalonner la migration des protéines.

Le gel est alors utilisé pour un électrotransfert des protéines puis coloré au bleu de Coomassie.

### - Electrotransfert des protéines sur membrane de nitrocellulose :

En fin d'électrophorèse, la membrane, les feuilles de papier Wattman et les éponges de mousse (Scotch Brite, 3M) sont équilibrés pendant 10 min dans le tampon de transfert froid (Tris 25 mM pH 8,3, glycine192 mM, méthanol 20% (v/v)). Le sandwich est réalisé en semi-immersion: la membrane et le gel sont superposés et encadrés de part et d'autre d'un papier et d'une éponge. L'ensemble, placé dans la cuve, est immergé dans le tampon de transfert , et les protéines sont transférées du gel (cathode) vers la membrane de nitrocellulose (anode) électrophorétiquement à 100 V pendant 45 min, à 4°C et sous agitation magnétique.

### - Immunodétection des protéines transférées :

Le matériel antigénique utilisé dans notre étude est de source bovine. Ne disposant pas d'anticorps primaires dirigés contre du matériel bovin, notre choix c'est porté sur des anticorps polyclonaux présentant une immunoréactivité croisée avec différentes espèces (homme, souris et rat).

### . Tampons utilisés :

Tampon TBS (Tris Buffer Saline): Tris-HCl 10 mM pH 8.0, NaCl 150 mM
Tampon TTBS: 0.05% de Tween 20 dans du TBS
Tampon de dilution de l'anticorps: BSA 1% dans du TTBS
Tampon de saturation des membranes : : lait 10% dans du TTBS

### . Révélation des protéines :

### Réaction antigénique.

Après électrophorèse des protéines et transfert, la membrane de nitrocellulose est colorée au rouge ponceau pour repérer les pistes de migration des protéines, puis décolorée. Les sites de liaisons non spécifiques de la membrane sont bloqués par une incubation dans le tampon de saturation adéquat pendant 60 min à température ambiante sous agitation douce (ou alternativement à 4°C pendant la nuit). La membrane est lavée trois fois (15 min puis 5 et 5 min) dans du tampon TTBS avant d'être incubée pendant 2 heures avec l'anticorps primaire anti caspase 10 humaine (1/2000) dilué dans le tampon de dilution. Une seconde série de lavages dans du TTBS précède la deuxième incubation (1 heure 30) avec l'anticorps secondaire anti-lapin couplé à la peroxydase de raifort (1/6000). La membrane est de nouveau lavée dans du TTBS puis rincée 5 min dans du TBS avant d'être révélée à l'ECL.

### Révélation à l'ECL.

La membrane est séchée rapidement sur du papier absorbant, incubée avec la solution de détection pour chimiluminescence du kit ECL pendant 1 min puis enveloppée dans du papier film Saran. Elle est alors exposée à un film autoradiographique pendant une durée de 8 min et les films sont développés.

### c) Résultats.

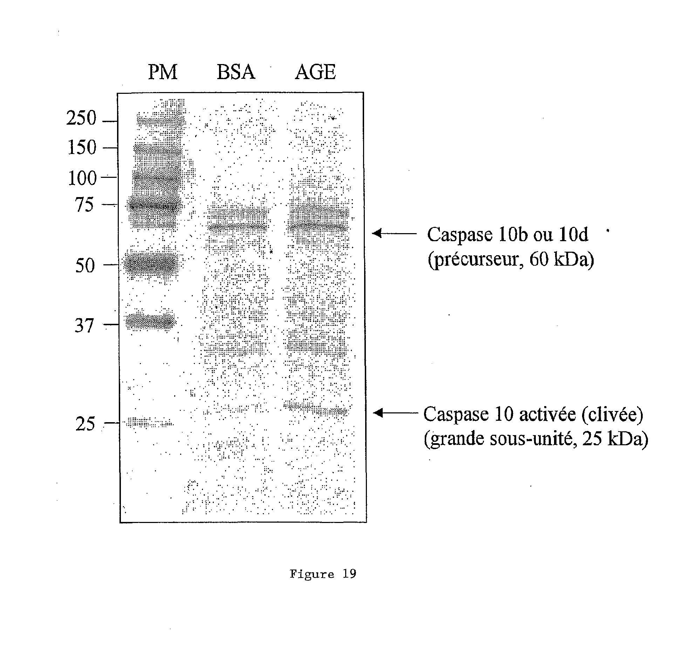

Ces résultats confortent les précédents précédemment où uniquement des peptides inhibiteurs de caspases avaient été utilisés. Les présents résultats illustrés par la figure 19 montre par analyse directe avec un anticorps spécifique que la grande sous-unité (25 kDa) de la caspase 10 activée à partir de son précurseur (caspase 10b ou 10d) est bien présente dans les péricytes incubés avec les AGE suggérant qu'il y a bien activation de la caspase 10 lors de l'apoptose des péricytes rétiniens induite par les AGE.

### 5) Conclusion.

L'effet protecteur du peptide z-VAD-fmk (inhibiteur à large spectre de caspases) sur l'apoptose des péricytes met en évidence l'activation de caspases dans la voie de transduction des signaux apoptotiques. Dans les péricytes cultivés avec ce peptide inhibiteur, la production de céramides et de DAG induite par les AGE-méthylglyoxal est bloquée, ceci suggère ainsi l'implication d'une caspase initiatrice (caspases-2, -8, -10), contrôlant la production des céramides et des DAG.

Dans un premier temps, nous avons vérifié si la caspase-8 initiatrice est l'enzyme activé en réponse aux AGE-Méthylglyoxal. Une incubation des cellules avec son inhibiteur spécifique z-IETD-fmk ne permet pas de bloquer l'apoptose. La caspase-8 ne semble donc pas être impliquée dans l'apoptose des péricytes.

Afin de définir si la caspase-2 initiatrice participe à la transduction du signal menant à l'apoptose, nous avons incubé les péricytes en présence du peptide inhibiteur z-VDVAD-fmk, spécifique de cette caspase. Le peptide z-VDVAD-fmk inhibe partiellement l'apoptose induite par les AGE-méthylglyoxal, suggérant que la caspase-2 initiatrice semble être impliquée dans l'apoptose des péricytes.

Nous avons également analysé les effets du peptide z-AEVD-fmk, décrit comme un inhibiteur de la caspase-10 initiatrice et inhibiteur moins spécifique de la caspase-8 initiatrice et de la caspase-9 effectrice. L'effet protecteur de ce peptide sur l'apoptose des péricytes suggère l'activation de la caspase-10 et/ou de la caspase-9, l'implication de la caspase-8 étant exclue par l'absence d'effet de z-IETD-fmk sur l'apoptose. Le peptide z-LEHD-fmk, inhibiteur décrit comme spécifique de la caspase-9 effectrice, a lui aussi un effet protecteur sur l'apoptose des péricytes, mais sans affecter la surproduction des DAG ou des céramides induite par les AGE-méthylglyoxal. Ce résultat suggère que la caspase-9, bloquée par z-LEHD-fmk, intervient en aval de la production des DAG et des céramides.

Ainsi, les caspases-2, 9 et 10 semblent participer à la transduction du signal apoptotique dans les péricytes rétiniens.

L'effet protecteur du peptide z-DEVD-fmk sur l'apoptose des péricytes suggère que les deux voies métaboliques proposées convergent vers une même caspase de la famille des CPP32-like protéases. Un traitement des péricytes avec ce peptide inhibiteur n'a pas affecté la surproduction des céramides et des DAG induite par les AGE-méthylglyoxal. Ce résultat permet alors de conclure que la (les) caspase(s) bloquée(s) par z-DEVD-fmk intervient(nent) en aval des céramides et des DAG. D'autre part, l'inhibition de l'apoptose observée en présence de z-DEVD-fmk ne semble pas être liée à l'inhibition des caspases-2, -8, -10 initiatrices puisque le niveau de production des céramides et des DAG n'a pas été affecté. De même, l'inhibition de l'apoptose par z-LEHD-fmk, spécifique de la caspase-9, ne semble pas liée à l'inhibition des caspases initiatrices, puisque le niveau de DAG et céramides n'a pas été affecté. Nous pouvons donc conclure que les deux voies métaboliques convergent bien vers deux caspases effectrices, la caspase 9 et la caspase-3, et peut-être la caspase 6 et 7 des CPP32-like protéases, intervenant dans la phase d'exécution de l'apoptose.

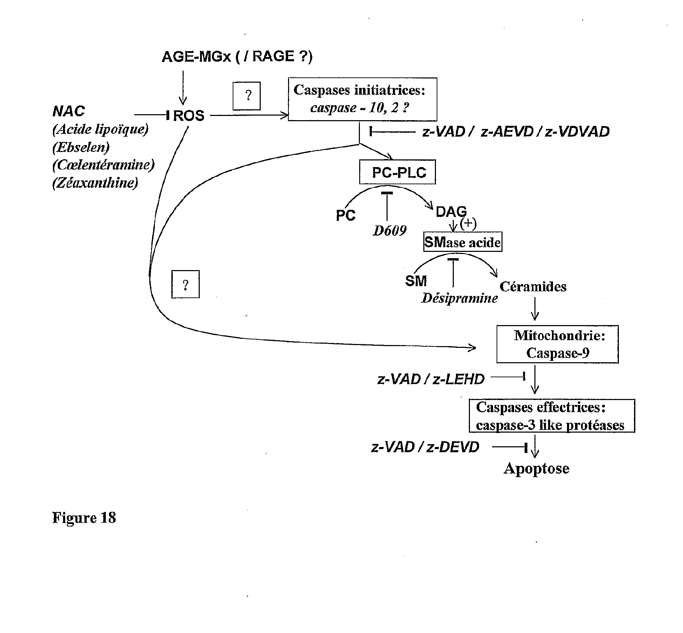

La figure 18 montre une voie de signalisation menant à l'apoptose des péricytes. Les AGE-méthylglyoxal induisent un stress oxydant qui semble activer une caspase initiatrice (la caspase-2 ou -10). L'activation de cette caspase activerait une cascade enzymatique impliquant dans un premier temps une phosphatidylcholine-phospholipaseC, puis une sphingomyélinase acide et ensuite la caspase 9 et une caspase effectrice des CPP32-like protéases. Il a été possible de déterminer que ces caspases 9 et 3 se situent au carrefour des deux voies métaboliques. En revanche, il n'est pas possible de conclure si les deux voies divergent en aval de la caspase initiatrice ou si le stress oxydant reste au carrefour de ces deux voies.

Les travaux décrits réalisés dans le cadre de l'invention mettent clairement en évidence qu'une caspase initiatrice, probablement la caspase 10 est impliquée dans la cascade d'événements biochimique menant à l'apoptose des péricytes induite par les AGE. Cette implication est étayée par des arguments expérimentaux basés sur une approche purement pharmacologique utilisant des inhibiteurs peptidiques spécifiques de différentes caspases. La figure 19 montre en que la caspase 10 est bien activée lorsque les péricytes rétiniens bovins sont soumis aux AGE. En effet, par immunodétection sur nitrocellulose à l'aide d'un anticorps spécifique qui reconnaît la forme clivée et active (grande sous-unité , 25 kDa) de la caspase 10 à partir de son précurseur (caspase 10b (FADD like interleukin-1-converting enzyme-2, FLICE-2) ou 10d, 60 kDa), il a été possible de détecter la grande sous-unité de la caspase activée (25 kDa) dans les cellules traitées par les AGE alors qu'elle est quasi inexistante dans le cas des cellules traitées par la BSA contrôle (voir Figure 19) bien que la quantité de précurseur (caspase 10b ou 10d) soit similaire dans les deux conditions (voir Figure 1).

### RÉFÉRENCES BIBLIOGRAPHIQUES

1) Aiello LP, Gardner TW, King GL, Blankenship G, Cavallerano JD, Ferris FL et Klein R (1998). Diabetic retinopathy. Diabetes care 21: 277-293
2) Algvere PV, Gouras P et Dafgard Kopp E (1999). Long-term outcome of RPE allografts in non-immunosuppressed patients with AMD. Eur. J. Ophthalmol. 9: 217-230
3) Alnemri ES (1999). Hidden powers of the mitochondria. Nature Cell. Biology 1: 40-42
4) Alnemri ES, Livingston DJ, Nicholson DW, Salvesen G, Thornberry NA, Wong WW et Yuan J (1996). Human ICE/CED-3 Protease Nomenclature. Cell 87: 171
5) Andrieu N, Salvayre R et Levade T (1994). Evidence Against Involvement of the acid lysosomal Sphingomyelinase in the TNF and Interleukin-1-induced Sphingomyelin Cycle and Cell Proliferation in Human Fibroblasts. Biochem. J. 303: 341-345
6) Ansari NH, Zhang W, Fulep E et Mansour A (1998). Prevention of pericyte loss by trolox in diabetic rat retina. J. Toxicol. Environm. Health 54: 467-478
7) Beisswenger PJ, Makita Z, Curphey TJ, Moore LL, Smith J, Brinck-Johnsen T, Bucala R et Vlassara H (1995). Formation of Immunochemical Advanced Glycosylation End Products Precedes and Correlate with Early Mannifestations of Renal and Retinal Diseases in Diabetes. Diabetes 44: 824-829
8) Bierhaus A, Chevion S, Chevion M, Hofmann M, Quehenberger P, Illmer T, Luther T; Berentshtein E, Tritschler H, Muller M, Wahl P, Ziegler R et Naworth P (1997). Advanced glycation end product-induced activation of NF-κB is suppressed by α-lipoic acid in cultured endothelial cells. Diabetes 46: 1481-1490
9) Bligh EG et Dyer WJ (1959). A rapid method of total lipid extraction and purification. Can. J. Biochem. Physiol. 37: 911-917
10) Brett J, Schmidt AM, Yan SD, Zou YS, Weidman E, Pinsky D, Nowygrod R, Neeper M, Przysiecki C, Shaw A, Mighelli A et Stern D (1993). Survey of the distribution of a newly characterized receptor for advanced glycation end products in tissues. Am. J. Pathol. 143: 1699-1712
11) Chibber R, Molinati PA, Rosatto N, Lambourne B et Kohner EM (1997). Toxic action of Advanced Glycation End Products on Cultured Retinal Capillary Pericytes and Endothelial Cells: Relevance to Diabetic Retinopathy. Diabetologia 40: 156-164
12) Cogan DG, Toussaint D et Kuwabara T (1961). Retinal vascular patterns IV. Diabetic retinopathy. Arch. Ophthalmol. 60: 100-112
13) DCCT - The Diabetes Control Complications Trial Research Group. The effects of intensive treatment of diabetes on the development and progression of long-term complications in insulin-dependent diabetes mellitus. N Engl J Med 1993, vol 239, p 977-986
14) Degenhardt TP, Thorpe SR et Baynes JW (1998). Chemical modification of Proteins by Methylglyoxal. Cell. Mol. Biol. 44: 1139-1145
15) Desai SD et Blanchard J (1998). In vitro evaluation of pluronicF127-based controlled-release ocular delivery systems for pilocarpine. J. Pharm. Sci. 87: 226-230
16) Duan H, Chinnaiyan AM, Hudson PL, Wing JP, He WW et Dixit VM (1996a). ICE-LAP3, a novel Mammalian Homologue of Caenorhabditis elegans Cell Death Protein Ced-3 is Activated During Fas- and TNF-induced Apoptosis. J. Biol. Chem. 271: 1621-1624
17) Duan H, Orth K, Chinnaiyan AM, Poirier GG, Froelich CJ, He WW et Dixit WM (1996b). ICE-LAP6, a Novel Member of the ICE/Ced-3 Gene Family, is Activated by Cytotoxic T Cell Protease Gramzyme B. J. Biol. Chem. 271: 16720-16724
18) Early Treatment Diabetic Retinopathy Study Research group : grading diabetic retinopathy from stereoscopic color fundus photographs : an extension of the modified Airlie House classification : ETDRS Report n°10, 1991 Ophthalmology, vol 98, p786-791
19) Fernandes-Alnemri T, Armstrong RT, Krebs J, Srinivasula SM, Wang L, Bullrich F, Fritz LC, Trapani JA, Tomaselli KJ, Litwack G et Alnemri ES (1995). In vitro Activation of CPP32 and Mch3 by Mch4, a Novel Apoptotic Cysteine Protease Containing Two FADD-like Domains. Proc. Acad. Natl. Sci. USA 93: 7464-7469
20) Frank RN Diabetic retinopathy. Chapter 1 in Progress in Retinal and Eye Research Vol 14 No. 2 Elsevier Science Ltd (Great Britain), 1995, p 361-392
21) Freedman KA, Klein JW et Crosson CE (1993). Beta-cyclodextrins enhance bioavailability of pilocarpine. Curr. Eye Res. 12: 641-647
22) Friedenwald JS (1950). Diabetic retinopathy Am. J. Ophthalmol. 33: 1187-1199
23) Friederich RL (1974). The pilocarpine Ocusert: a new drug delivery system. Ann. Ophthalmol. 6: 1279-1284
24) Garcia-Calvo M, Peterson EP, Leiting B, Ruel R, Nicholson DW et Thornberry NA (1998). Inhibition of Human Caspases by Peptide-based and Macromolecular Inhibitors. J. Biol. Chem. 273: 32608-32613
25) Genestier L, Prigent A-F, Paillot R, Quemeneur L, Durand I, Banchereau J, Revillard JP et Bonnefoy-Bérard N (1998). Caspase-dependant ceramide Production in FAS- and HLA Class I-mediated Peripheral T Cell Apoptosis. J. Biol. Chem. 273: 5060-5066
26) Gluzman Y, Reichl H et Solnick D (1982) in Eukaryotic Viral Vectors (Gluzman Y, ed) pp 187-192, Cold Spring Harbor Laboratory, Cold Spring Harbor, New York
27) Graham FL, Smiley J, Russel WC et Nairn R (1977). Characteristics of a human cell line transformed by DNA from human adenovirus type 5. J. Gen. Virol. 36: 59-74
28) Grange JD (1995). La rétinopathie diabétique. Paris, Milan, Barcelone: Masson: 632
29) Hammes HP, Martin S, Federlin K, Geisen K et Brownlee M (1991). Aminoguanidine Treatment inhibits the Development of Experimental Diabetic Retinopathy. Proc. Natl., Acad. Sci. USA 88: 11555-11558
30) Hangai M, Tanihara H, Honda Y et Kaneda Y (1998). In Vivo delivery of phosphorothioate oligonucleotides into the murine retina. Arch. Ophthalmol. 116: 342-348
31) Jaffrezou JP, Chen G, Duran GE, Muller C, Bordier C, Laurent G, Sikic BI et Levade T (1995). Inhibition of lysosomal acid sphingomyelinase by agents which reverse multidrug resistance. Biochim. Biophys. Acta. 1266: 1-8
32) Kahn HA et Hiller R (1974). Blindness caused by diabetic retinopathy. Am. J. Ophthalmol. 78: 58-67
33) Karesh JW (1987). Polytetrafluoroethylene as a graft material in ophthalmic plastic and reconstructive surgery. An experimental and clinical study. Ophthal. Plast. Reconstr. Surg. 3: 179-185
34) Kuwabara T et Cogan DG (1963). Retinal vascular patterns VI. Mural cells of the retinal capillaries. Archs. Ophthal. 69: 492-502
35) Lai CM, Shen WY, Constable I et Rakoczy PE (2000). The use of adenovirus-mediated gene transfer to develop a rat model for photoreceptor degeneration. Invest. Ophthalmol. Vis. Sci. 41: 580-584
36) Lander HM, Tauras JM, Ogiste JS, Hori O, Moss RA, et Schmidt AM (1997). Activation of the receptor for advanced glycation end products triggers a p21ras-dependent mitogen-activated protein kinase pathway regulated by oxidant stress. J. Biol. Chem. 272: 17810-17814
37) Leahey AB, Gottsch JD et Syark WJ (1993). Clinical experience with N-butyl cyanoacrylate (Nexacryl) tissue adhesive. Ophthalmol. 100: 173-180
38) Lecomte M, Paget C, Ruggiero D, Wiernsperger N, et Lagarde M (1996). Docosahexaenoic acid is a major n-3 polyunsaturated fatty acid in bovine retinal microvessels. J. Neurochem. 66: 2160-2167
39) Leeds JM, Henry SP, Bistner S, Scherill S, Williams K et Levin AA (1998). Pharmacokinetics of an antisense oligonucleotide injected intravitreally in monkeys. Drug Metab. Dispos. 26: 670-675
40) Li W, Liu X, Yanoff M, Cohen S et Ye X (1996). Cultured Retinal Capillary Pericytes Die by Apoptosis After an Abrupt Fluctuation from High to Low Glucose Levels: a Comparative Study With Retinal Capillary Endothelial Cells. Diabetologia 39: 537-547
41) Li W, Liu X, He Z, Yanoff M, Jian B et Ye X (1998). Expression of Regulatory genes by Retinal Pericytes After Rapid Glucose Reduction. Invest. Ophtamol. Vis. Sci.39: 1535-1543
42) Li W, Yanoff M, Jian B et He Z (1999). Altered mRNA levels of antioxidant enzymes in pre-apoptotic pericytes from human diabetic retinas. Cell. Mol. Biol. (Noisy-le-grand) 45: 59-66
43) Li W, Shen S, Khatami M, et Rockey JH (1984). Stimulation of retinal capillary pericyte protein and collagen synthesis in culture by high glucose concentration. Diabetes 33: 785-789
44) Li W, Yanoff M, Liu X et Ye X (1997). Retinal Capillary Pericytes Apoptosis in Early Diabetic Retinopathy. Chin. Med. J (Engl) 110: 659-663
45) Lo TWC, Westwodd ME, McLellan AC, Selwoll T, et Thornalley PJ (1994). Binding and modification of proteins by methylglyoxal under physiological conditions. A kinetic and mechanistic study with Nα-acetylarginine, Nα-acetylcysteine, Nα-acetyllysine, and bovine serum albumin. J. Biol. Chem. 269: p32299-32305
46) Maillard LC. Action des acides aminés sur les sucres : formation des mélanoïdines par voie méthodique 1912. C R Hebd Sci, Acad Sci Paris, vol 154, p 66-68
47) Mandarino LJ, Sundarraj N, Finlayson J et Hassel JR (1993). Regulation of fibronectin and laminin synthesis by retinal capillary endothelial cells and pericytes in vitro. Exp. Eye Res. 57: 609-621
48) Mathias S, Pena LA et Kolesnick RN (1998). Signal transduction of stress via ceramides. Biochem. J. 335: 465-480
49) McLellan AC, Thornalley PJ, Benn J et Sonksen PH (1994). Glyoxalase System in Clinical Diabetes Mellitus and Correlation with Diabetic Complications. Clin. Sci. 7: 21-29
50) Mizutani M, Kern TS et Lorenzy M (1996). Accelerated Death of Retinal Microvascular Cells in Human and Experimental Diabetic. Retinopathy. J. Clin. Invest. 97: 2883-2890
51) Müller-Decker K, Doppler C, Amtmann E et Sauer G (1988). Interruption of Growth Signal Transduction by an Antiviral and Antitumoral Xanthate compound. Exp. Cell. Res. 177: 295-302
52) Müller-Decker K (1989). Interruption of TPA-induced Signals by an Antiviral and Antitumoral Xanthate Compound: Inhibition of a Phospholipase C-type Reaction. Biochem. Biophys. Res. Com. 162: 198-205
53) Nathan DM, Fogel HA, Godine JE et al. (1991). Role of diabetologist in evaluating diabetic retinopathy. Diabetes Care 14: 26-33
54) Neeper M, Schmidt AM, Brett J, Yan SD, Wang F, Pan YC, Elliston K, Stern D et Shaw A (1992). Cloning and expression of a cell surface receptor for advanced glycosylation end products of proteins. J. Biol. Chem. 267: 14498-15004
55) Nicholson DW, Ali A, Thornberry NA Vaillancourt JP, Dink CK, Gallant M, Gareau Y, Griffin PR, Labelle M, Lazebnick YA, Monday NA, Raju SM, Smulson NE, Yamin TT, Yu VL et Müller-Decker K (1995). Identification and Inhibition of the ICE/CED-3 Protease Necessary for Mammalian Apoptosis. Nature 376: 37-43
56) Nishikawa T, Edelstein D, Hu XL, Yamagishi SI, Matsumura T, Kaneda Y, Yorak MA, Beebe D, Dates PJ, Hammes PJ, Giardino I et Brownlee M (2000). Normalizing mitochondrial superoxide production blocks three pathways of hyperglycaemic damage. Nature 404: 787-790
57) Nunez G, Benedict MA, Hu Y et Inohara N (1998). Caspases: the proteases of the apoptotic pathway. Oncogene 17: 3237-3245
58) Nuyts RM, Kooijman-DeGroot MJ, Prins et Pels E (1999). Use of a polyurethane patch for temporary closure of a sterile corneal perforation. Arch. Ophtalmol. 117: 1427-1429
59) Oganesian A, Gabrielian K, Ernest JT et Patel SC (1999). A new model of retinal pigment epithelium transplantation with microspheres. Arch. Ophthalmol. 117: 1192-1200
60) Okasaki T, Kondo T, Kitano T et Tashima M (1998). Diversity and Complexity of Ceramides Signaling in Apoptosis. Cell. Signal. 10: 685-692
61) Paget C, Lecomte M, Ruggiero D, Wiernsperger N et Lagarde M (1998). Modification of Enzymatic Antioxidants in Retinal Microvascular by glucose or Advanced Glycation End Products. Free. Radic. Biol. Med. 25: 121-129
62) Palmberg PF (1977). Diabetic retinopathy. Diabetes 26: 703-709
63) Podesta F, Romeo G, Liu WH, Krajewski S, Reed JC, Gerhardinger C et Lorenzy M (2000). Bax is Increased in the Retina of Diabetic Subjects and in Associated With Pericytes Apoptosis In Vivo and In Vitro. Am. J. Pathol. 156: 1025-1032
64) Porta M, Molinatti PA, Dosso AA, Williams FMK, Brooks RA et Kohner EM (1994). Growth of bovine retinal pericytes and endothelial cells in high glucose concentrations. Diabetes and Metabolism 20: 25-30
65) Preiss J, Loomis CR, Bishop WR, Stein R, Niedel JE et Bell RM (1986). Quantitative measurement of sn-1,2-diacylglycerols present in platelets, hepatocytes, and ras- and sis-transformed normal kidney cells. J. Biol. Chem. 261: 8597-8600
66) Rubin AL, Stenzel KH, Miyata T, White MJ et Dunn M (1973). Collagen as a vehicle for drug delivery. Preliminary report J. Clin. Pharmacol. 13: 309-312
67) Ruggiero-Lopez D, Rellier N, Lecomte M, Lagarde M et Wiernsperger N (1997). Growth Modulation of Retinal Microvascular Cells by Early and Advanced Glycation Products. Diabetes Res. Clin. Pract. 34: 135-142
68) Schaffner W et Weissmann C (1973). A rapid, sensitive and specific method for the determination of protein in dilute solution. Anal. Biochem. 50: 502-504
69) Schalkwijck CG, Ligtvoet N, Twaalfhoven H, Jager A, Blaauwgeers HGT, Schlingemann RO, Tarnow L, Parving HH, Stehouwer CDA et van Hinsbergh VWM (1999). Amadori Albumin in Type I diabetic patients. Correlation with markers of endothelial function, association with diabetic nephropathy, and localization in retinal capillaries. Diabetes 48: 2446-2453
70) Schmidt AM, Vianna M, Gerlach M, Brett J, Ryan J, Kao J, Esposito C, Hegarty H, Hurley W, Clauss M, Wang F, Pan YCE, Tsang TC et Stern D (1992). Isolation and characterization of two binding proteins for advanced glycosylation end products from bovine lung which are present on the endothelial cell surface. J. Biol. Chem. 267: 14987-14997
71) Schuchman EH, Suchi M, Takahashi T, Sandhoff K et Desnick RJ (1991). Human acid sphingomyelinase. Isolation, nucleotide séquence and expression of the full-length and alternatively spliced cDNAs. J. Biol. Chem. 266: 8531-8539.
72) Schütze S, Potthoff K, Machleidt T, Berkovic D, Wiegmann K et Krönke M (1992). TNF Activates NF-kB by Phosphatidylcholine-specific Phospholipase C-Induced "Acidic" Sphingomyelin Breakdown. Cell. 71: 765-776
73) Shinohara M;, Thornalley PJ, Giardino I, Beisswenger P, Thorpe SR, Onorato J et Brownlee M (1998). Overexpression of glyoxalase-I in bovine endothelial cells inhibits intracellular advanced glycation endproduct formation and prevents hyperglycemia-induced increases in macromolecular endocytosis. J. Clin. Invest. 101: 1142-1147
74) Soulis T, Thallas V, Youssef S, Gilbert RE, McWilliam BG, Murray-McItosh RP et Cooper ME (1997). Advanced glycation end products and their receptors co-localise in rat organs susceptible to diabetic microvascular injury. Diabetologia 40: 619-628
75) Stennicke HR et Salvesen GS (1998). Properties of the caspases. Biochim. Biophys. Acta. 1387: 17-31
76) Stitt AW, Li YM, Gardiner TA, Bucala R, Archer DB et Vlassara H (1997). Advanced Glycation End Products (AGEs) Co-localize With AGE Receptors in the Retinal Vasculature of Diabetic and AGE-infused Rats. Am. J. Pathol. 150: 523-535
77) Su T et Gillies MC (1992). A simple method for the in vitro culture of human retinal capillary endothelial cells. Invest. Ophthalmol. Vis. Sci. 33: 2809-2813
78) Thornalley PJ (1998). Cell activation by glycated proteins. AGE receptors, receptor recognition factors and functional classifications of AGEs. Cell. Mol. Biol. (Noisy-le-grand) 44: 1013-1023
79) Thornalley PJ (1999). The clinical significance of glycation. Clin. Lab. 45: 261-273
80) Thornberry NA, Rano TA, Peterson ER, Rasper DM, Timkey T, Garcia-Calvo M, Houtzager VM, Nordstrom PA, Roy S, Vaillancourt JP, Chapman KT et Nicholson DW (1997). A combinatorial Approach Defines Specificities of Members of the Caspase Family and Granzymes B. J. Biol. Chem. 272: 17907-17911
81) UKPDS(a)- UK Prospective Diabetes Study Group. Intensive blood-glucose control with sulphonylureas or insulin compared with conventional treatment and risk of complications in patients with type 2 diabetes (UKPDS 33) Lancet 1998, vol 352 (9131), p 837-853
82) UKPDS(b)- UK Prospective Diabetes Study Group. Effect of intensive blood-glucose control with metformin on complications in overweight patients with type 2 diabetes (UKPDS 34) Lancet 1998, vol 352 (9131), p 854-865
83) Vandervoort J et Ludwig A (1999). Evaluation of pilocarpine loaded gelatin particles for topical ophthalmic use. J. Pharm. Belg. 54: 85-86
84) Vlassara H, Li Y-M, Imani F, Wojciechowicz D, Yang Z, Liu FT et Cerami A (1995). Identification of galectin-3 as a high-affinity binding protein for advanced glycation end products (AGE): a new member of the AGE-receptor complex. Mol. Med. 1: 634-646
85) Wang E, Norred WP, Bacon CW, Riley RT et Mirrill AH Jr (1991). Inhibition of Sphingolipid Biosynthesis by Fumonisins. Implication for Diseases Associated with Fusarium Monoliforme. J. Biol. Chem. 266: 14486-14490
86) Westwood ME et Thornalley PJ (1995). Molecular characteristics of methylglyoxal-modified bovine and human serum albumins. Comparison with glucose-derived advanced glycation endproducts-modified serum albumins. J. Protein Chem. 14: 359-372
87) Wilkins MR, Occleston NL, Kotecha A, Waters L et Khaw PT (2000). Sponge delivery variables and tissues levels of 5-fluorouracil. Br. J. Ophthalmol. 84: 92-97
88) Yamagishi S, Hsu CC, Taniguchi M Harada S, Yamamoto Y, Ohsawa K, Kobayashi K et Yamamoto H (1995). Receptor mediated Toxicity to Pericytes of Advanced Glycosylation End Products: a Possible Mechanism of Pericyte Loss in Diabetic Microangiopathy. Biochem. Biophys. Res. Commun. 213: 681-687
89) Yan SD, Schmidt AM, Anderson GM, Zhang J, Brett J, Zou YS, Pinsky D et Stern D (1994). Enhanced cellular oxidant stress by the interaction of advanced glycation end producys with their receptors/binding proteins. J. Biol. Chem. 269: 9889-9897
90) Yang BZ, Makita Z, Horii Y, Brunelle S, Cerami A, Sehajpal P, Suthanthiran M et Vlassara H (1991). Two novel rat liver membrane proteins that bind advanced glycosylation end products: relationship to macrophage receptor for glucose-modified proteins. J. Exp. Med. 174: 515-524
91) Zhu G, Mallery SR et Schwendeman SP (2000). Stabilization of proteins encapsulated in injectable poly(lactide-co-glycolide). Nature Biotechnol. 18: 52-57
92) Zuckert WR, Marquis H et Goldfine H (1998). Modulation of enzymatic activity and biological function of Listeria monocytogenes broad-range phospholipase C by amino acid substitution and by replacement with the Bacillus cereus ortholog. Infect. Immun. 66: 4823-4831
93) Zurowska-Pryczkowska K, Sznitowska M et Janicki S (1999). Studies on the effect of pilocarpine incorporation into a submicron emulsion on the stability of the drug and the vehicle. Eur. J. Pharm. Biopharm. 47: 255-260

## Revendications

1. Inhibiteur de l'apoptose des péricytes rétiniens pour la prévention ou le traitement de la rétinopathie diabétique, ledit inhibiteur de l'apoptose des péricytes rétiniens étant un inhibiteur de caspase.

2. Inhibiteur selon la revendication 1, **caractérisée en ce que** l'inhibiteur de caspase est de type peptidique.

3. Inhibiteur selon la revendication 2, **caractérisée en ce que** l'inhibiteur de caspase est un peptide modifié chimiquement par un groupement benzyleoxycarbonyle (z) du côté N-terminal et par un groupe (O-méthyle)-fluorométhyle cétone (fmk) du côté C-terminal.

4. Inhibiteur selon l'une quelconque des revendications 1 à 3, **caractérisée en ce que** l'inhibiteur de caspase est le tripeptide z-VAD-fmk.

5. Inhibiteur selon l'une quelconque des revendications 1 à 3, **caractérisée en ce que** l'inhibiteur de caspase est un inhibiteur d'une caspase initiatrice ou un inhibiteur d'une caspase effectrice.

6. Inhibiteur selon la revendication 5, **caractérisée en ce que** l'inhibiteur de caspase est un inhibiteur d'une caspase initiatrice choisie parmi les caspases 2 ou 10 ou un inhibiteur d'une caspase effectrice choisie parmi les caspases 3 ou 9.

7. Inhibiteur selon la revendication 6, **caractérisée en ce que** l'inhibiteur de caspase initiatrice 10 est le peptide z-AEVD-fmk.

8. Inhibiteur selon la revendication 6, **caractérisée en ce que** l'inhibiteur de caspase initiatrice 2 est le peptide z-VDVAD-fmk.

9. Inhibiteur selon la revendication 6, **caractérisée en ce que** l'inhibiteur de caspase effectrice 3 est le peptide z-DEVD-fmk.

10. Inhibiteur selon la revendication 6, **caractérisée en ce que** l'inhibiteur de caspase effectrice 9 est le peptide z-LEHD-fmk.

11. Composition pharmaceutique comprenant à titre d'agents actifs au moins un inhibiteur de l'apoptose de péricytes rétiniens tels que définis dans l'une quelconque des revendications précédentes.

12. Composition pharmaceutique selon la revendication 11, **caractérisée en ce qu'**elle comprend en outre à titre d'agents actifs un composé antioxydant et/ou un inhibiteur de la phosphatidylcholine-phospholipase C et/ou un inhibiteur d'une sphingomyélinase acide.

13. Composition pharmaceutique selon l'une des revendications 11 ou 12, **caractérisée en ce que** le dosage en agent (s) actif (s) permet une administration journalière comprise entre 0,01 et 200 mg/kg de masse corporelle, préférentiellement entre 0,5 et 75 mg/kg de masse corporelle et tout préférentiellement entre 1 et 50 mg/kg de masse corporelle.

14. Composition pharmaceutique selon l'une des revendications 11 à 13, **caractérisée en ce qu'**elle comprend de 5% à 95% (w/w) et de préférence de 20 % à 80 % (w/w) d'agent (s) actif (s).

15. Composition pharmaceutique selon l'une des revendications 11 ou 12, **caractérisée en ce qu'**elle se présente sous une forme pour une administration topique ou une injection oculaire et/ou **caractérisée en ce qu'**elle comprend entre 0,01 et 10% et de préférence entre 0,01 et 4% en poids d'agent (s) actif (s).


SEQUENCE LISTING

<110>  MERCK SANTE

<120>  UTILISATION D'INHIBITEURS DE L'APOPTOSE DES PERICYCLE POUR LE
       TRAITEMENT ET/OU LA PREVENTION DE LA RETINOPATHIE DIABETIQUE

<130>  BIP119721EPPC01

<140>  EP 11151829.6
<141>  2011-01-24

<160>  9

<170>  PatentIn version 3.5

<210>  1
<211>  4
<212>  PRT
<213>  Artificial Sequence

<220>
<223>  Inhibiteur spécifique de la caspase-3

<400>  1

Asp Glu Val Asp
1


<210>  2
<211>  4
<212>  PRT
<213>  Artificial Sequence

<220>
<223>  Inhibiteur spécifique de la caspase-9

<400>  2

Leu Glu His Asp
1


<210>  3
<211>  4
<212>  PRT
<213>  Artificial Sequence

<220>
<223>  Inhibiteur spécifique de la caspase-8

<400>  3

Ile Glu Thr Asp
1


<210>  4
<211>  5
<212>  PRT
<213>  Artificial Sequence

<220>
<223>  Inhibiteur spécifique de la caspase-2

<400>  4

Val Asp Val Ala Asp
1               5


<210>  5
<211>  4
<212>  PRT
<213>  Artificial Sequence

<220>
<223>  Inhibiteur spécifique de la caspase-10

<400>  5

Ala Glu Val Asp
1


<210>  6
<211>  16
<212>  PRT
<213>  Artificial Sequence

<220>
<223>  séquence hydrophobe du peptide signal du facteur de croissance
       des fibroblastes de Kaposi

<400>  6

Ala Ala Val Ala Leu Leu Pro Ala Val Leu Leu Ala Leu Leu Ala Pro
1               5                   10                  15


<210>  7
<211>  27
<212>  DNA
<213>  Artificial Sequence

<220>
<223>  Oligomère de désoxyribonucléotides codant corespondant à la
       séquence du pepetide AEVD


<220>
<221>  misc_feature
<222>  (6)..(6)
<223>  r is a or g

<220>
<221>  misc_feature
<222>  (7)..(8)
<223>  n is a, c, g, or t

<220>
<221>  misc_feature
<222>  (14)..(14)
<223>  n is a, c, g, or t

<220>
<221>  misc_feature
<222>  (17)..(17)
<223>  r is a or g

<220>
<221>  misc_feature
<222>  (20)..(20)
<223>  n is a, c, g, or t

<220>
<221>  misc_feature
<222>  (23)..(23)
<223>  y is t or c

<400>  7
gatccrnnat ggcngargtn gaytaag                                          27


<210>  8
<211>  26
<212>  DNA
<213>  Artificial Sequence

<220>
<223>  Oligomère de désoxyribonucléotides non-codant corespondant à la
       séquence du pepetide AEVD


<220>
<221>  misc_feature
<222>  (9)..(9)
<223>  r is a or g

<220>
<221>  misc_feature
<222>  (12)..(12)
<223>  n is a, c, g, or t

<220>
<221>  misc_feature
<222>  (15)..(15)
<223>  y is t or c

<220>
<221>  misc_feature
<222>  (18)..(18)
<223>  n is a, c, g, or t

<220>
<221>  misc_feature
<222>  (24)..(25)
<223>  n is a, c, g, or t

<400>  8
aattcttart cnacytcngc catnng                                           26


<210>  9
<211>  5
<212>  PRT
<213>  Artificial Sequence

<220>
<223>  site de clivage protéolitique des caspases


<220>
<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<400>  9

Xaa Asp Xaa Xaa Asp
1               5